(19) Europäisches Patentamt
European Patent Office
Office européen des brevets

(11) EP 1 813 947 A1

(12) **EUROPEAN PATENT APPLICATION**
published in accordance with Art. 158(3) EPC

(43) Date of publication:
**01.08.2007 Bulletin 2007/31**

(21) Application number: **05799253.9**

(22) Date of filing: **27.10.2005**

(51) Int Cl.:
***G01N 33/543*** *(2006.01)* ***G01N 33/53*** *(2006.01)*
***G01N 33/541*** *(2006.01)*

(86) International application number:
**PCT/JP2005/019787**

(87) International publication number:
**WO 2006/046644 (04.05.2006 Gazette 2006/18)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.10.2004 JP 2004314639**
**25.02.2005 JP 2005052003**

(71) Applicant: **SANKO JUNYAKU CO., LTD.**
**Tokyo 101-0032 (JP)**

(72) Inventors:
- **Takayama, Shigeo**
**Toride-shi, Ibaraki 3020015 (JP)**
- **Yamada, Yuji**
**Tsukuba-shi, Ibaraki; 3050045 (JP)**

(74) Representative: **Grünecker, Kinkeldey, Stockmair & Schwanhäusser Anwaltssozietät**
**Maximilianstrasse 58**
**80538 München (DE)**

(54) **METHOD OF EXAMINING ALZHEIMER S DISEASE AND DIAGNOSTIC REAGENT**

(57) The present invention is intended to devise a process of measuring β-amyloid in a biological sample such as blood and to apply the process to diagnosis of Alzheimer's disease.

It is possible to assay Alzheimer's disease by measuring a total amount of β-amyloid 1-42 and β-amyloid 1-42 fragments each of which retains a C-terminal site of the β-amyloid 1-42 in the biological sample by an immunological assay in which an antibody which recognizes the C-terminal site of β-amyloid 1-42 is used. It is preferable that the immunological assay be a competitive immunological assay.

**FIG.11**

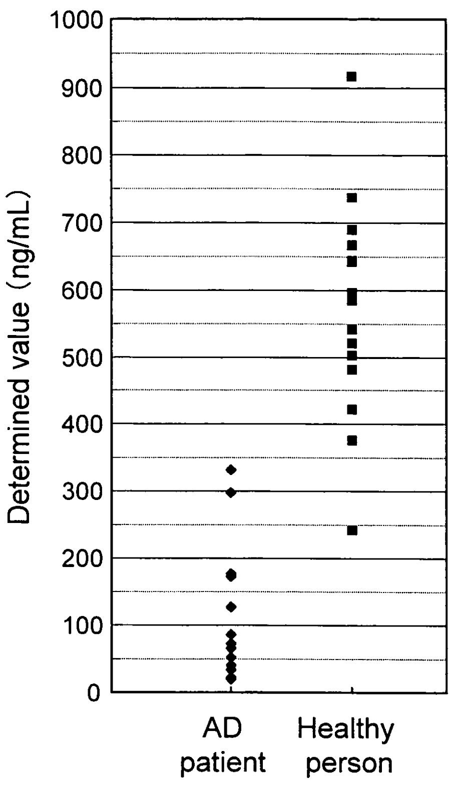


EP 1 813 947 A1

## Description

Technical Field

The present invention relates to diagnostic methods of diseases caused by β-amyloid, such as Alzheimer's disease, by measuring a total amount of β-amyloid 1-42 and β-amyloid 1-42 fragments each of which retains a C-terminal site of β-amyloid 1-42 in a biological sample, especially in blood, and to diagnostic reagents therefor.

Background Art

β-Amyloid (hereinafter, referred to as "Aβ") is a main constitutional component of the characteristic amyloid plaque which is seen in a brain of a patient with Alzheimer's disease (AD) and it is known that Aβ is produced by a β-secretase action which cleaves a β-position of an N-terminal site of a precursor protein thereof (APP) and by a γ-secretase action of preselinin which cleaves an APP-C-terminal site which is present in a cell membrane.
Aβ molecular species are known to have various molecular weight sizes but the most well known of those species in connection with neurotoxicity are an Aβ species composed of 40 amino acids (hereinafter, referred to as "Aβ1-40") and an Aβ species composed of 42 amino acids (hereinafter, referred to as "Aβ1-42"). Aβ1-42 has a nature which readily forms fibers and it is known that Aβ1-42 is deposited in the early stages of AD and forms an amyloid plaque.
As described above, the deposition of Aβ is a pathological characteristic which is characteristic of the brain of an AD patient and the Aβ is also found in a cerebrospinal fluid and in the blood. It has been reported that the Aβ1-42 concentration in the cerebrospinal fluid is low in AD patients (Non-patent Documents 1 to 4). Further, there are reports that the Aβ1-42 which is present in plasma of familial AD patients increases and that the Aβ1-40 or Aβ1-42 concentration in the plasma of sporadic AD patients is increased (Non-patent Documents 5 and 6), while on the other hand it has also been reported to be virtually the same as in the group of people in normal health (Non-patent Documents 7 to 9) and the actual behavior of the Aβ is unclear.
The measurement of Aβ in biological samples in the past has been carried out mainly using a double antibody sandwich measurement method. Suzuki et al. have reported the preparation of a plurality of monoclonal antibodies with different recognition sites with various Aβ peptide fragments as immunogens and a sandwich measurement method in which those antibodies are used in combination (Patent Document 1). It is disclosed that the Aβ in the cerebrospinal fluid of an AD patient is measured with this method and that Aβ1-40 is the main component, and that, in a formic acid extract of an AD patient's brain, Aβ1-40, Aβ1-42, and Aβ3-42 are the main components.
There are also reports in which competitive immunological assays have been used. In Non-patent Document 18, the Aβ in a serum of hyperlipidaemia patients was measured by a competitive immunological assay using rabbit polyclonal antibodies specific to Aβ1-40 and it is disclosed that Aβ1-40 tends to increase with advancing age. Further, Graham Paul et al. have disclosed the preparation of a monoclonal antibody which is specific to Aβ1-42 and which does not react with Aβ1-40 and Aβ1-43 and indicated that the antibody can accomplish immuno-tissue staining of the plaques formed of blood vessel amyloids, fibrous amyloids, or the like of AD patient's brain tissue (Patent Document 2).
Double antibody ELISA reagents for determining the Aβ concentration in cerebrospinal fluid are commercially available from two companies, but although values determined with ELISA reagents are correlated, they are quite different from one another (Non-patent Document 10). Differences in clinical samples which serve as subjects for measurement, an antibody which is used for the measurement, a method for the measurement, sensitivity of the method for the measurement, and a method by which the Aβ1-42 which is used for a calibration curve has been produced, for example, can be cited as reasons for the difference in the determined values.
Further, in cases where the sample for the clinical investigation is plasma or serum it is known that interfering substances which interact with Aβ molecules are present in the plasma or serum and those substances must be removed or isolated in advance (Non-patent Documents 11 to 13). However, in those cases where plasma or serum is used as the sample for a clinical investigation, the actual method used to remove those interfering substances, measurement errors due to the removal process, dilution effects, a titer of the antibody which is being used, and the measurement sensitivity, for example, are all liable to have a great effect on the measured value.
Aβ in the cerebrospinal fluid can be measured by means of a general measurement procedure, for example, by the two antibody sandwich method of measurement using antibodies which bind specifically to Aβ, and the measurement of Aβ1-42 in particular has been recognized as being clinically useful (Non-patent Documents 1, 3, 4, 7, and 15 to 17). However, the use of cerebrospinal fluid as a sample involves subjecting the patient to a high risk of physical load or physical function impairment when the sample is being collected and in practice cerebrospinal fluid is not being used as a sample at the present time.
Usually, blood samples (serum or plasma) are considered to be most general and suitable samples for in vitro diagnostic with a low risk of physical impairment. However, Aβ in the serum is almost impossible to be detected by using the general measurement methods such as the double antibody sandwich measurement method which had been carried out in the past and the clinical usefulness of such measurements has not been discovered (Non-patent Document 7). It is though that this is because the amount of Aβ which has migrated from the cerebrospinal fluid into the blood is very small.
The amount of Aβ1-42 in the cerebrospinal fluid of an AD patient tends to be lower than that in a healthy person but the amount of Aβ1-42 in the blood still cannot be recognized as being constant and it is very difficult to use the blood in a pathological or diagnostic study of AD.

Patent Document 1: WO94/17197
Patent Document 2: WO96/25435
Non-patent Document 1: Tamaoka A, et al., J. Neurol. Sci. 1997; 148:41-45
Non-patent Document 2: Andreasen N, et al., Arch. Neurol. 1999; 56:673-680
Non-patent Document 3: Galasko D, et al., Arch. Neurol. 1998; 55:937-945
Non-patent Document 4: Motter R, et al., Ann. Neurol. 1995; 38:643-648
Non-patent Document 5: Mayeux R, Ann. Neurol. 1999; 46:412-416
Non-patent Document 6: Mehta PD, Arch. Neurol. 2000; 57:100-105
Non-patent Document 7: Tamaoka A, et al., J. Neurol. Sci. 1996; 141:65-68
Non-patent Document 8: Vanderstichele H, et al., Amyloid 2000; 7:245-258
Non-patent Document 9: Fukumoto H, et al., Arch. Neurol. 2003; 60:958-964
Non-patent Document 10: Suzuki N, et al., Science 1994; 264:1336-1340
Non-patent Document 11: Chan W, et al., Biochemistry 1996; 35:7123-1230
Non-patent Document 12: Biere AL, et al., J. Biol. Chem. 1996; 271:32916-32922
Non-patent Document 13: Kuo YM et al., Biochem. Biophys. Res. Commun. 2000; 268:750-756
Non-patent Document 14: Naniwa et al., An investigation of sensitive immunological assays with electrochemiluminescence (ECL), JJCLA, 1996, Vol.21, No.5
Non-patent Document 15: Tero T, et al., Neurobiology of Aging 21(2000) 735-740
Non-patent Document 16: Kanai M, et al., Ann Neurol 1998; 44:17-26
Non-patent Document 17: Schröder J, et al., Mol Psychiatry 1997; 2:505-7
Non-patent Document 18: Christopher C.T. Smith et al., Neuroscience Letters, 362(2004) 48-50
Non-patent Document 19: G. Zhu et al., J. Pharm. Biomed. Anal. 24, 281-290 (2000)

Disclosure of the Invention

Problems to be solved by the Invention

The present invention is based upon an understanding of the problems outlined above and applies to the diagnosis of Alzheimer's disease by means of a method for the measurement of Aβ in the blood of AD patients.

Means for solving the Problems

The inventors of the present invention have carried out thorough research with a view to resolving the above-mentioned problems. First, the inventors of the present invention investigated whether or not Aβ1-42 in the serum of AD patients could be measured with good sensitivity by using the double antibody sandwich measurement method in which the electrochemiluminescence method (Non-patent Document 14) which has a high sensitivity-wide measuring range capacity is used. The antibody 21F12 (produced by Innogenetics Inc.) which is specific to a C-terminal site of Aβ1-42 was used as a primary antibody and an antibody which was specific to a 1-5 amino acid site of Aβ1-42 was used as a second antibody and the Aβ1-42 was measured in the serum of AD patients and of healthy people. The results showed that the sensitivity was quite good as compared with the conventional methods but no significant difference could be seen between the amounts of Aβ1-42 in the serum of the AD patients and in the serum of healthy people (see, Experiment Examples 1 and 2 and FIG. 4).

Here, in order to verify the stability of Aβ1-42 synthetic peptide in the serum, the inventors of the present invention confirmed that the Aβ1-42 content fell with the passage of time when Aβ1-42 synthetic peptide and serum were both present. Further, it was confirmed that when the Aβ1-42 synthetic peptide was present along with serum no fall in the Aβ1-42 content occurred with the passage of time if a protease inhibitor was introduced into the serum beforehand (see, Experiment Example 3 and FIG. 5). This fact signifies that Aβ is degraded and fragmented rapidly by protease in biological fluids such as cerebrospinal fluid and blood, for example, and that the reason why Aβ cannot be measured is not that only trace amounts migrate into the blood, and it suggests that precise measurement of full length Aβ1-42 in blood for example is impossible because of the fact that it is fragmented.

The finding suggests that Aβ1-42 migrated into blood from the cerebrospinal fluid is degraded therein in a short period of time, and that degradation of Aβ1-42 proceeds also during storage of the serum sample. Thus, this is the main reason that the accurate measurement cannot be attained. In other words, the precise measurement of Aβ1-42 cannot be achieved by the double antibody sandwich measurement method because the Aβ1-42 is fragmented in blood.
However, although the Aβ1-42 is degraded and the content falls, the degraded Aβ1-42 fragments are still present. Thus, when measuring Aβ1-42 which has been treated with a protease by the two measuring methods, that is, by the competitive immunological assay using antibody which recognizes the C-terminal site of Aβ1-42 and by the above-mentioned electrochemiluminescence double antibody sandwich measurement method, it could hardly be detected at all by the double antibody sandwich measurement method (see, Experiment Example 4 and FIG. 2) but it was confirmed that a value close to the initial amount of Aβ1-42 was obtained by the competitive immunological assay (see, Experiment Example 5 and FIG. 3). That is, it was first discovered that the Aβ1-42 content could be measured precisely by measuring the total amount of Aβ1-42 and Aβ1-42 fragments which retained the C-terminal site of Aβ1-42 (hereinafter, the total amount is referred to as "Aβx-42") by the competitive immunological assay.

Next, the results obtained on measuring Aβx-42 in the serum of AD patients and healthy people showed a significant difference there between and the possibility of the use of the serum for the diagnosis of AD was confirmed, and the present invention is achieved based upon those discoveries.
The method of assaying AD by the competitive immunological assay, which is one of the present inventions, is an excellent method with which Aβx-42 can be detected with high efficiency without the need for a procedure of removing the substances which interact with Aβ1-42 in plasma and serum. In the past, there had been reports of competitive immunological assays in which antibodies which recognize Aβ1-40 were used (Non-patent Document 18), but there had been no disclosure concerning the competitive immunological assay in which an antibody which recognizes just the C-terminal site of Aβ1-42 like that of the present invention or any report of its effect and, moreover, the facts that the method of the present invention has an excellent effect of reflecting the Aβ1-42 content in blood precisely and that this is useful for the diagnosis of AD were first discovered by the inventors of the present invention.

Moreover, the fact that measuring Aβx-42 can be useful in the diagnosis of AD was also first discovered by the inventors of the present invention, so the method of measuring Aβx-42 is not limited to the above-mentioned competitive immunological assay, and detection by the surface plasmon resonance method using an antibody which is specific to the C-terminal site of Aβ1-42 (Non-patent Document 19), a method of measurement where peptides which retains the C-terminal sites of Aβ1-42 are isolated by means of affinity chromatography using an antibody which is specific to the C-terminal site of Aβ1-42, and the like can be used. The essence of the present invention is that AD can be diagnosed by measuring Aβx-42.

The present invention is an invention where the usefulness of the measurement of Aβx-42 with an antibody which recognizes the C-terminal site of Aβ1-42 has been confirmed, but the usefulness of the measurement of Aβ1-x in the same way has also been confirmed. Aβ1-x signifies the total amount of fragments of various Aβ isotypes which retains the N-terminal site of Aβ, such as Aβ1-40 and Aβ1-37. By using antibodies which recognize the N-terminal site of Aβ in a competitive assay method of the present invention, it is possible to measure those Aβ isotypes at once irrespective of the type. Specifically, measurement was carried out by the competitive immunological assay using monoclonal antibody 3D6 (produced by Innogenetics Inc.) prepared by immunizing with Aβ1-5, or monoclonal antibody 6E10 (produced by Chemicon International, Inc.) prepared by immunizing with Aβ1-17. The results showed no significant difference between the measured values obtained with serum from AD patients and those obtained with serum from healthy people. This shows the importance of measuring specifically the Aβx-42 fragments in the blood for the diagnosis of AD.
According to those facts, the present invention reflects the amount of Aβ1-42 in blood precisely by measuring Aβx-42, and moreover, the clinical usefulness is confirmed between the serum samples of AD patients and healthy people, thereby enabling AD to be diagnosed using a blood sample. Further, there is a possibility that it could be used for the diagnosis of diseases where Aβ is one of the causes.

That is, according to the present invention, there is provided a method of assaying Alzheimer's disease, which includes measuring a total amount of β-amyloid 1-42 and β-amyloid 1-42 fragments each of which retains a C-terminal site of the β-amyloid 1-42 in a biological sample by means of an immunological assay in which an antibody specific to the C-terminal site of the β-amyloid 1-42 is used.
Among the β-amyloid 1-42 fragments, a β-amyloid 1-42 fragment which retains the C-terminal site of β-amyloid 1-42 specifically indicates a peptide which retains the C-terminal site of β-amyloid 1-42, and indicates a β-amyloid 1-42 fragment which preferably retains 1 or more amino acid residues, preferably 2 or more amino acid residues, and more preferably 3 or more amino acid residues from the C-terminal of β-amyloid 1-42. It is desirable that the β-amyloid 1-42 fragment which retains the C-terminal site of β-amyloid 1-42 preferably bind to an antibody specific to the C-terminal site of Aβ1-42. Hereinafter, the β-amyloid 1-42 fragment is sometimes referred to as "Aβ1-42 C-terminal site-retaining peptide", and β-amyloid 1-42 and the Aβ1-42 C-terminal site-retaining peptide may sometimes be referred to as "Aβx-42" as a whole.

It is preferable that the immunological assay be a competitive immunological assay. Procedures for the competitive immunological assay can be performed by, for example, two types of the following procedures in which a peptide which retains C-terminal of Aβ1-42 and which is immobilized as a solid phase or an immobilized antibody is used.
A first aspect of the method of the present invention by the competitive immunological assay includes the following steps (a) and (b) of:

(a) reacting a support on which a peptide which retains a C-terminal site of β-amyloid 1-42 is immobilized as a solid phase, a biological sample, and an antibody labeled with a labeling substance (antibody which recognizes the C-terminal site of β-amyloid 1-42); and
(b) determining, after washing, Aβx-42 by measuring an amount of a labeled antibody which is bound to the peptide which retains the C-terminal site of the β-amyloid 1-42 and which is immobilized as a solid phase.
In addition to the above-mentioned steps, the first aspect may further include the following step of:
(c) assaying the occurrence or absence of Alzheimer's disease by comparing a measured value of Aβx-42 in a biological sample with that in a biological sample from a healthy person.

A second aspect of the method of the present invention by the competitive immunological assay includes the following steps (a) and (b) of:

(a) reacting a support on which an antibody which recognizes a C-terminal site of Aβ1-42 is immobilized as a solid phase, a biological sample, and a peptide which retains the C-terminal site of Aβ1-42 and which is labeled with a labeling substance; and
(b) determining, after washing, Aβx-42 by measuring an amount of a labeled peptide which retains the C-terminal site of Aβ1-42 and which is bound to the immobilized antibody.
In addition to the above-mentioned steps, the second aspect may further include the following step of:
(c) assaying the occurrence or absence of Alzheimer's disease by comparing a measured value of Aβx-42 in a biological sample with that in a biological sample from a healthy person.
A synthetic peptide can be used for the labeled Aβ1-42 C-terminal site-retaining peptide but Aβ1-42 C-terminal site-retaining peptide composites (hereinafter, referred to as "peptide composites") in which a carrier substance has been bound can also be used. The term carrier substance signifies a compound which can be labeled with a labeling substance such as a ruthenium complex and these include, for example, polylysine (poly-L-lysine), dextran, bovine serum albumin, and polypeptides which retain many free amino groups. The carrier substance can be added to any position in the peptide as long as binding property between the peptide and the antibody is not impaired, and it is desirable that the carrier substance be preferably added to an N-terminal site of the peptide.

The AD diagnostic reagent (kit) of the present invention is a diagnostic reagent for Alzheimer's disease which has an antibody which recognizes the C-terminal site of Aβ1-42 as an essential structural component and with which Aβx-42 can be measured. The diagnostic reagent has a constitution which differs according to the immunological assay used, but in the case of the competitive immunological assay, either an Aβ1-42 C-terminal site-retaining peptide which is immobilized as a solid phase or an antibody which is immobilized as a solid phase is used. In the case where an Aβ1-42 C-terminal site-retaining peptide which is immobilized as a solid phase is used, an antibody which is labeled with a labeling substance forms another constituent component, and in the case where an antibody which is immobilized as a solid phase is used, an Aβ1-42 C-terminal site-retaining peptide which is labeled with a labeling substance forms another constituent component. A peptide composite in which a carrier substance is bound can also be used for the labeled Aβ1-42 C-terminal site-retaining peptide. The carrier substance may be, for example, polylysine, dextran, bovine serum albumin, or a polypeptide which retains many free amino groups. The carrier substance can be added to any position in the peptide as long as binding property between the peptide and the antibody is not impaired, and it is desirable that the carrier substance be preferably added to an N-terminal site of the peptide.
Polyclonal antibodies or monoclonal antibodies can be used for the above-mentioned antibody in the methods and reagents for diagnosis of the present invention. Further, the above-mentioned antibodies are preferably antibodies which recognize Aβ1-42 but do not recognize Aβ1-40, and more preferably mouse monoclonal antibodies each of which is specific to the C-terminal site of Aβ1-42 and obtained by using as an immunogen a 33-42 amino acid site of the β-amyloid 1-42. Specific examples thereof include 21F12 (produced by Innogenetics Inc.), AB5078P (produced by Chemicon International, Inc.) and 8G7 (produced by Nanotools GmbH).
It is preferable that the above-mentioned biological sample be serum or plasma.
Examples of the labeling substance include fluorescent substances, enzymes, pigments, and luminescent substances, with a ruthenium complex being preferable. The support for immobilization is preferably magnetic beads.
The method of measuring β-amyloid 1-42 of the present invention includes measuring a total amount of β-amyloid 1-42 and β-amyloid 1-42 fragments each of which retains a C-terminal site of the β-amyloid 1-42 in a biological sample by means of an immunological assay in which an antibody specific to the C-terminal site of the β-amyloid 1-42 is used. It is preferable that the biological sample be serum or plasma.

Effects of the Invention

The present invention enables AD to be diagnosed by measuring Aβx-42 in a biological sample using an immunological assay in which an antibody which recognizes specifically the C-terminal site of Aβ1-42 is used. According to the present invention, it is not necessary to remove substances that interact with Aβ1-42 in serum or plasma, and Aβx-42 can be detected accurately and efficiently.

Brief Description of the Drawings

FIGS. 1(a) to 1(c) are schematic explanatory drawings showing a first example of a method of assaying Alzheimer's disease of the present invention.
FIGS. 2(a) to (c) are schematic explanatory drawings showing a second example of a method of assaying Alzheimer's disease of the present invention.
FIG. 3 is a graph showing results of Experiment Example 1.
FIG 4 is a graph showing results of Experiment Example 2.
FIG. 5 is a graph showing results of Experiment Example 3.
FIGS. 6(a) and 6(b) are schematic explanatory drawings showing metabolism of Aβ1-42 in blood.
FIG. 7 is a graph showing results of Experiment Example 6 and 7.
FIG. 8 is a graph showing results of Example 1.
FIG. 9 is a graph showing results of Example 2.
FIG. 10 is a graph showing a calibration curve obtained using a standard substance of Example 3.
FIG. 11 is a graph showing the results of Example 3.
FIG. 12 is a graph showing a calibration curve obtained using a standard substance of Experiment Example 8.
FIG. 13 is a photograph showing results of electrophoresis of Experiment Example 8.
FIGS. 14(a) to 14(d) are charts showing first results of Experiment Example 9.
FIG. 15 is a graph showing first results of Example 9.
FIG. 16 is a graph showing a calibration curve obtained in Experiment Example 9.
FIG. 17 is a chart showing the second results of Experiment Example 9.
FIG. 18 is a chart showing the results of Experiment Example 10.

Description of Reference Numerals

10, 20: support, magnetic beads, 12, 24: Aβ1-42 synthetic peptide, 13: Aβ1-42 synthetic peptide-binding support, Aβ1-42 synthetic peptide-binding magnetic beads, 14, 22: antibody specific to C-terminal of Aβ1-42, 16, 26: labeling substance, 17: labeled antibody, 23: Aβ1-42 antibody-binding support, Aβ1-42 antibody-binding magnetic beads, 27: labeled Aβ1-42 synthetic peptide, 30: Aβx-42 in biological sample, 32: reaction cup, 34: reaction solution, 40: luminescence of labeling substance

Best Mode for carrying out the Invention

Hereinafter, embodiments of the present invention are described, but of course, various modifications can be made as long as there is no deviation from the technical concept of the present invention.
The method of assaying Alzheimer's disease of the present invention is intended for assaying the presence or absence of morbidity of Alzheimer's disease by measuring a total amount (amount of Aβx-42) of Aβ1-42 and Aβ1-42 fragments each of which retains a C-terminal site of Aβ1-42 in a biological sample by an immunological assay in which an antibody specific to the C-terminal site of Aβ1-42.
The immunological assay to be used in the present invention is not particularly limited as long as Aβx-42 in a biological sample can be measured. Any one of, for example, a competitive immunological assay, detection by the surface plasmon resonance method (Non-patent Document 19), a measurement method involving isolating a peptide which retains the C-terminal of Aβ1-42 by affinity chromatography, and the like can be used, with the competitive immunological assay being preferable.
Examples of the biological sample include serum, plasma, cerebrospinal fluid, bone marrow fluid, tissue or cell extracts, and cell culture media, with the serum and plasma being preferable.
The antibody to be used in the present invention can be either a monoclonal antibody or a polyclonal antibody as long as it is an antibody which is specific to the C-terminal site of Aβ1-42. An antibody which reacts with Aβ1-42 but does not react with Aβ1-40 is preferable, and commercially-available examples thereof include 21F12 (produced by Innogenetics Inc.), AB5078P (produced by Chemicon International, Inc.), and 8G7 (produced by Nanotools BmbH) which are mouse monoclonal antibodies each of which is specific to the C-terminal site of Aβ1-42 and obtained by using as an immunogen a 33-42 amino acid site of the Aβ1-42, with the 21F12 being preferable. The antibodies which do not react with Aβ1-40 include those antibodies which, even though they have a weak reactivity, have essentially no effect in the measurement of the total amount of the Aβ1-42 C-terminal site. Further, it makes no difference if there is reactivity with Aβ1-43.

The antibodies used in the present invention can be prepared using the usual methods.

The preparation of a monoclonal antibody involves using a peptide which retains the C-terminal site of Aβ1-42 as antigen, producing a composite with a carrier protein as required and immunization by inoculating this into an animal. The antibody producing cells obtained from the spleen or lymph nodes of the above-mentioned immunized animal are fused with myeloma cells and the hybridomas which produce antibodies which exhibit strong specificity for the C-terminal site of Aβ1-42 can be prepared by selection. The procedure should be carried out in accordance with the existing known methods.

Aβ1-42 can also be used as the immunizing antigen, but since the target antibodies are antibodies which are specific to the C-terminal site of Aβ1-42, peptides each of which retains the C-terminal site of Aβ1-42, such as Aβ33-42, can be selected appropriately. Generally, a composite with a carrier protein is used for the antigen, and these can be prepared using various coupling agents, such as glutaraldehyde, carbodiimide, and maleimide active esters. Bovine serum albumin, thyroglobulin, hemocyanin, and the like can be used for the carrier protein and generally, methods involving coupling in a proportion of 1 to 5 times by weight are used.

The animal which is immunized may be a mouse or a guinea pig, for example, and the inoculation can be carried out subcutaneously, intramuscularly, or intraperitoneally. In administration, it may be administered as a mixture with complete Freund's adjuvant or incomplete Freund's adjuvant, and the administration is generally carried out once per 2 to 5 weeks. The antibody producing cells obtained from the spleen or lymph nodes of the immunized animal fused with myeloma cells and isolated as hybridomas. Myeloma cells originating from mice, rats, humans, or the like, can be used for the myeloma cells, and cells originating from the same species as the antibody producing cells are preferable, but there are cases where cells originating from different species can be used.

The cell fusion operation can be carried out using a known method, for example the Kayler and Millstein method (Nature, 256, 495-497 (1975)). Polyethylene glycol and Sendai virus, for example, can be cited as fusion promoting agents and the cell fusion can be carried out generally by reacting the antibody producing cells and the myeloma cells in a ratio generally of 1:1 to 1:10 for a period of about 1 to 10 minutes using a 20 to 50% concentration of polyethylene glycol (average molecular weight 1000 to 4000) at a temperature of 20 to 40°C, and preferably of 30 to 37°C.

The screening for the hybridomas which produce antibodies which have specificity for the C-terminal site of Aβ1-42 can be carried out using various immunochemical methods. For example, use can be made of the ELISA method, the Western Blot method, or the competitive method. Further, antibodies which react with Aβ1-42 and do not react with Aβ1-40 can be selected by using the Aβ1-42 peptide and the Aβ1-40 peptide.

Cloning is then carried out by means of the limiting dilution method, for example, from the wells which have been selected in this way and the target clones are obtained. Generally, the selection and growth of the hybridomas is carried out in an animal cell culture medium (for example RPMI1640) to which hypoxanthine, aminopterin, and thymidine (HAT) have been added and which contains from 10 to 20% bovine fetal serum. The clones obtained in this way are transplanted into the abdominal cavities of BALB/C mice to which Britstan has been administered beforehand and ascetic fluid which contains a high concentration of the monoclonal antibody is collected after 10 to 14 days and this can be used as the raw material for antibody purification. Further, the clones can be cultured and the culture liquid can be used as the raw material for antibody refinement. The recovery of the monoclonal antibodies should be achieved using a known method for the refinement of immunoglobulins and, for example, it can be achieved using a means such as ammonium sulfate fractionation, PEG fractionation, and ethanol fractionation, using an anion exchange material, or using affinity chromatography.

Polyclonal antibodies can also be prepared using the usual methods. They can be prepared by inoculating an antigen into an animal such as a rabbit or guinea pig as a composite by the same procedure as described above using a peptide which has the C-terminal site of Aβ1-42 as its principal structure. Polyclonal antibodies can be obtained through refinement by the methods described above by measuring the antibody potency after appropriate collection and using the serum with the highest potency as the raw material for the refinement of the antibody.

Hereinafter, the method of assaying Alzheimer's disease of the present invention is described by referring to an example in which the competitive immunological assay is used as the immunological assay.

Descriptions are made of the method of the present invention which uses the competitive immunological assay. The competitive immunological assay to be used in the present invention uses an antibody specific to the C-terminal site of Aβ1-42 and an Aβ synthetic peptide, and uses as a reaction basis the competitive binding reaction of the Aβ1-42 which retains the C-terminal of Aβ1-42 and the Aβ synthetic peptide to the antibody in a biological sample such as blood.

Any kind of the Aβ synthetic peptide can be used in the method of the present invention as long as the peptide reacts with the antibody which recognizes the C-terminal site of Aβ1-42. Specifically, peptides each of which retains the C-terminal site of Aβ1-42 can be used. Examples of the peptides each of which retains the C-terminal site of Aβ1-42 include, but not limited to, synthetic peptides such as Aβ33-42 and Aβ17-42 in addition to the Aβ1-42 that is a full-length peptide. The peptides can be synthesized by the normal method such as a solid phase synthetic method. Hereinafter, an example in which an Aβ1-42 synthetic peptide is used as the Aβ synthetic peptide is described in detail.

FIGS. 1(a) to 1(c) are schematic explanatory drawings showing a first example of the method of assaying Alzheimer's disease of the present invention. In the example, the immunological assay is performed by the competitive immunological assay in which a peptide which retains a C-terminal site of Aβ1-42 is immobilized as a solid phase on a support.

In FIGS. 1(a) to 1(c), reference numeral 10 denotes a support, reference numeral 12 denotes an Aβ1-42 synthetic peptide, reference numeral 32 denotes a reaction vessel (reaction cup), and reference numeral 34 denotes a reaction solution (for example, a buffer solution). As shown in FIG. 1(a), the Aβ1-42 synthetic peptide 12 is allowed to bind (immobilized as a solid phase) to the support 10 to prepare an Aβ1-42 synthetic peptide-binding support 13.

Materials for the support 10 may be glass, plastic (for example, polystyrene, polyamide, polyethylene, or polypropylene), metal, or the like. The support may take a form of a cup, a flat plate, particles, or the like with no particular limitation. It is preferable that the support 10 be magnetic microbeads (magnetic beads).

The immobilization of the Aβ synthetic peptide onto the support is performed according to the normal method. In a case where the magnetic beads are used as the support, it is preferable that the magnetic beads be allowed to react with the Aβ1-42 synthetic peptide in a buffer solution, treated with a blocking agent, and then preserved in the blocking agent. Hereinafter, descriptions are made of an example in which the magnetic beads are used as the support.

The Aβ1-42 synthetic peptide-binding magnetic beads 13 which has been prepared as described above is mixed with an antibody 17 (hereinafter, referred to as "labeled antibody"), which is obtained by labeling an antibody 14 specific to the C-terminal site of Aβ1-42 with the labeling substance 16, and a biological sample so that competitive reactions are allowed to proceed between the Aβ1-42 synthetic peptide 12 which is bound to the magnetic beads and Aβ1-42 in the biological sample, and between a fragment 30 which retains the C-terminal site of Aβ1-42 and the labeled antibody 17, to thereby competitively binding them (FIG. 1(b)).

The labeling substance 16 to be used in the present invention may be an enzyme, a luminescent substance, a fluorescent substance, an isotope, or the like, but not particularly limited. A ruthenium complex is preferable.

The method of preparing the labeling antibody is carried out in accordance with the normal methods. For example, the antibody and a ruthenium complex (Origen TAG-NHS Ester, produced by Igen Co.) are reacted in a buffer solution, and then, 2M of glycine is added thereto to cause further reaction. After that, the preparation can be achieved by purifying the labeled antibody by gel filtration column chromatography.

After the competitive reaction, the reaction solution is aspirated and the magnetic beads are washed, to thereby remove free substances which are unbound to the magnetic beads from the reaction solution (BF separation).

Next, the amount of the labeling substance bound to the magnetic beads is measured. The measurement of the labeling substance is performed by the normal method. For example, in a case where a ruthenium complex is used as the labeling substance, as shown in FIG. 1(c), the ruthenium complex-labeled antibody which is bound to the magnetic beads is allowed to emit light by applying thereto electrical energy under the presence of tryptophyl amine, and intensity of the ruthenium complex luminescence 40 is measured (Non-patent Document 14).

At this time, the amount of the labeled antibody bound to the bead-bound peptide decreases when a large amount of Aβx-42 is present in the sample owing to the competitive reaction between them. Thus, the luminescence intensity decreases as compared with that in a case where no competitive substance is present. By using the degree of the decrease in luminescence intensity (represented as inhibition rate (%) in the examples hereinbelow), the amount of Aβx-42 in the sample can be determined. Since the amount of Aβx-42 in a sample from a patient suffering from Alzheimer's disease is significantly lower than that in a sample from a healthy person, assay of Alzheimer's disease can be performed by measuring the amounts of Aβx-42 in the samples by the method of the present invention.

FIGS. 2(a) to 2(c) are schematic explanatory drawing showing a second example of the method of assaying Alzheimer's disease of the present invention. In the example, the immunological assay is performed by the competitive immunological assay in which an antibody that specifically recognizes the C-terminal site of Aβ1-42 is immobilized as a solid phase on a support.

In FIGS. 2(a) to 2(c), reference numeral 20 denotes a support, and reference numeral 22 denotes an antibody that specifically recognizes a C-terminal site of Aβ1-42. As shown in FIG. 2(a), the Aβ1-42 antibody 22 is allowed to bind (immobilized as a solid phase) to the support 20 that is a magnetic bead or the like to prepare the Aβ1-42 antibody-binding support 23. The immobilization of the antibody onto the support is performed according to the normal method. Hereinafter, descriptions are made of an example in which the magnetic beads are used as the support.

The antibody-binding magnetic beads prepared as described above is mixed with a labeled Aβ1-42 synthetic peptide obtained by labeling the Aβ1-42 synthetic peptide 24 with the labeling substance 26 and a biological sample to allow a competitive reaction to proceed, to thereby competitively binding them (FIG. 2(b)).

After the competitive reaction, the same operations as for the first example described above, and the amount of the labeling substance which has been bound to the magnetic beads is measured (FIG. 2(c)), whereby the amount of Aβx-42 in the sample can be determined.

The Aβ1-42 synthetic peptide to be labeled can be used as it is, but peptide composites each of which has a carrier substance bound to the Aβ1-42 can also be used. Here, the term carrier substance signifies a compound to which the labeling substance can be bound, and examples include polylysine (poly-L-lysine), dextran, bovine serum albumin and polypeptides which retain many free amino groups, and polylysine is preferable. The amount of the labeling substances per one Aβ1-42 peptide can be increased and a rise in sensitivity can be anticipated as a result of using a carrier substance and so the use of these composites is preferable.

The labeled peptide composite prepared by the method in which a carrier substance which is labeled with the labeling substance and the Aβ1-42 synthetic peptide are bound by means of, for example, a crosslinking agent. At this time, a spacer, such as a lysine peptide composed of 15 lysine units, for example, can be bound optionally on the N-terminal site of the Aβ1-42 beforehand, as required. Further, labeling with a labeling substance is possible after preparing the peptide composite, and both methods can be used optionally and no limitation is imposed.

A diagnostic reagent (kit) based on the immunological assay of the present invention includes as an essential constitutional component an antibody specific to a C-terminal site of Aβ1-42, and further includes as a constitutional component a synthetic peptide which retains the C-terminal site of Aβ1-42.

Types of the constitutional components of the reagent may appropriately be set depending on the kind of the immunological assay employed. For example, in the competitive immunological assay, as described above, it is preferable that a support (preferably magnetic beads) on which the synthetic peptide which retains the C-terminal site of Aβ1-42 is immobilized as a solid phase and a labeled antibody be used as the constitutional components, or a support (preferably magnetic beads) on which an antibody is immobilized as a solid phase and the synthetic peptide which retains the C-terminal site of Aβ1-42 be used as the constitutional components. The labeled synthetic peptide which retains the C-terminal site of Aβ1-42 preferably includes a constitutional component a peptide composite obtained by binding a carrier substance to the peptide which retains the C-terminal site of Aβ1-42 as described in the second example of the method of the present invention.

Note that, it is free to append a buffer, a measurement tool, or the like for the reaction to the reagent.

[Examples]

Hereinafter, the present invention is described in detail by means of examples, but the present invention is limited only to not exceeding the essential features and it is not limited by the illustrative examples below.

(Experiment Examples 1 and 2) Electrochemiluminescence double antibody sandwich measurement method

In Experiment Examples 1 and 2, analysis was performed by the electrochemiluminescence double antibody sandwich measurement method in which two types of antibodies which are specific to Aβ are used. A 21F12 mouse monoclonal antibody (produced by Innogenetics Inc.) which was an antibody which reacts specifically with Aβ1-42 prepared by immunizing a mouse with a 33-42 amino acid site of Aβ1-42 was used as a primary antibody, and a 3D6 mouse monoclonal antibody (produced by Innogenetics Inc.) which was prepared by immunizing a mouse with a 1-5 amino acid site of Aβ1-42 and labeled with a ruthenium complex was used as a secondary antibody.

The methods used to prepare the respective constitutional components of a reagent are described hereinbelow.

(1) Method of preparing 21F12 antibody-binding magnetic beads

A 21F12 mouse monoclonal antibody was diluted to an antibody concentration of 1 mg/ml with a 10 mmol/l potassium phosphate buffer solution (pH 7.8), and 0.5 ml of the antibody was mixed with 0.5 ml of magnetic beads (Dynabeads M-450 Epoxy, produced by Dynal Co.) having a concentration of 30 mg/ml. The liquid mixture was stirred for 16 hours at 25°C so that the antibody was bound to the magnetic beads. Then, only the liquid solution was removed from the magnetic bead solution to remove free antibodies which had not been bound to the magnetic beads and remained in the solution. Then, 1 ml of a 4% Block Ace reagent (produced by Dainippon Sumitomo Pharma Co., Ltd.) as a blocking agent was added to the antibody-binding magnetic beads, and the mixture was stirred for 3 hours at 25°C. Then, the magnetic beads were washed with 10 ml of the 4% Block Ace reagent (washing five times with 2 ml of the 4% Block Ace reagent). After washing, the 21F12 antibody-binding magnetic beads were mixed with 0.5 ml of the 4% Block Ace reagent and stored at 4°C until they were to be used.

(2) Method of preparing a ruthenium complex-labeled 3D6 antibody

A 3D6 mouse monoclonal antibody (produced by Innogenetics Inc.) was diluted to an antibody concentration of 1 mg/ml with a 10 mmol/l potassium phosphate buffer solution (pH 7.8). Then, 17.6 μl of a 10 mg/ml ruthenium complex (Origen TAG-NHS Ester, produced by Igen Co.) was added to 0.5 ml of the 1 mg/ml antibody, and the mixture was stirred for 30 minutes at 25°C. Then, 30 μl of 2 mol/l glycine was added thereto, and the mixture was stirred for 30 minutes at 25°C.

Then, the ruthenium complex-labeled antibody liquid was applied to gel filtration column chromatography (Sephadex G-25, manufactured by GE Healthcare Bio-Science KK) packed into a glass tube of diameter 1 cm and height 30 cm and the ruthenium-labeled antibodies were isolated from the non-labeling ruthenium complex and purified. Elution was carried out with a 10 mmol/l potassium phosphate buffer solution (pH 6.0).

(Experiment Example 1) Measurement of Aβ1-42 synthetic peptide

Seven 500-μl polystyrene cups (hereinafter, referred to as "reaction cups") were prepared, and 150 μl of a reaction solution containing 50 mmol/l Tris HCl, 1% BSA, 0.15 mol/l NaCl, 0.01% Tween 20, 10 mmol/l EDTA2Na, and 0.1% normal mouse serum (pH 7.5) (hereinafter, referred to as "sandwich measurement reaction solution") was poured into each of the reaction cups. 50 μl of a sample obtained by diluting the Aβ1-42 synthetic peptide with the sandwich measurement reaction solution to a concentration of 0, 0.5, 1, 5, 10, 25, 50, or 100 pg/ml was added to each of those reaction cups, respectively. Then, 25 μl of 21F12 antibody-binding magnetic beads which had been diluted to a concentration of 2 mg/ml with the sandwich measurement reaction solution was added to each of the reaction cups to allow a reaction to proceed for 9 minutes at 30°C (first reaction).

Subsequently, the magnetic beads were trapped with a magnet and the liquid was removed from the reaction cups. The magnetic beads were washed twice with 350 μl of a washing solution containing 50 mmol/l Tris HCl, 0.0 1 % (w/v) Tween 20, and 0.15 mol/l NaCl pH 7.5, and non-specifically-binding substances other than that of the antigen-antibody reaction was removed (BF separation).

Then, 200 μl of a ruthenium complex-labeled 3D6 antibody which had been diluted to a concentration of 4 μg/ml with the sandwich measurement reaction solution was added and reacted for 9 minutes at 30°C (second reaction). After the reaction, the magnetic beads were trapped with a magnet, and the liquid in the cups was removed. The magnetic beads were washed twice with 350 μl of a washing solution and the non-specifically-binding substances other than that of the antigen-antibody reaction were removed (BF separation).

Subsequently, 300 μl of tryptophyl amine was put into each cup, and mixed with the magnetic beads. The ruthenium complex emitted light when electrical energy was applied in this state, and the luminescence intensity was detected with a detector. Moreover, the measuring procedure after the addition of the magnetic beads to the reaction cups described above was carried out with an automated ruthenium luminescence measurement device Picolumi 8220 (manufactured by Sanko Junyaku Co., Ltd.). Table 1 and FIG 3 show the results. Note that, in FIG. 3, ECL values were represented by values each obtained by subtracting a measured value when the sample concentration is 0 from a measured value of the ruthenium complex luminescence intensity.

[Table 1]

| Measurement results of Experiment Example 1 | | |
|---|---|---|
| Sample concentration (pg/mL) (Aβ1-42 synthetic peptide) | ECL value | Difference from the blank |
| 0(Blank) | 469.9 | - |
| 0.5 | 1083.9 | 614.0 |
| 1 | 1835.9 | 1366.0 |
| 5 | 10805.4 | 10335.5 |
| 10 | 22857.5 | 22387.6 |
| 25 | 75796.0 | 75326.1 |
| 50 | 160254.4 | 159784.5 |
| 100 | 345037.0 | 344567.1 |
| ECL value: measured value of luminescence intensity of a ruthenium complex Difference from the Blank: ECL value - ECL value when a sample concentration is 0 | | |

As shown in Table 1 and FIG. 3, the electrochemiluminescence double antibody sandwich measurement method was confirmed to be a measurement system which enabled detection of the Aβ1-42 synthetic peptide in amounts up to 0.5 pg/ml. This is a measurement system with which a sensitivity more than ten times the highest sensitivity obtained with past reagents from among the Aβ1-42 sandwich measuring reagents generally known in the past.

(Experiment Example 2) Measurement of serum from AD patients and healthy people by the electrochemiluminescence double antibody sandwich measurement method

A plurality of reaction cups was prepared as required, and 150 μl of a sandwich measurement reaction solution was poured into each of the reaction cups. Samples (standard products for creation of calibration curve) obtained by diluting the Aβ1-42 synthetic peptide to 0, 0.5, 1, 5, 10, 25, 50 or 100 pg/ml with the sandwich measurement reaction solution and 50 μl samples of serum from 25 AD patients and from 25 healthy people were introduced respectively into the reaction cups and mixed. Then, 25 μl of 21F12 antibody-binding magnetic beads diluted with sandwich measurement reaction solution to a concentration of 2 mg/ml were added to each cup and reacted for 9 minutes at 30°C (first reaction). Subsequently, after trapping the magnetic beads with a magnet, the liquid was removed from the reaction cups and the magnetic beads were washed twice with 350 μl of a washing solution containing 50 mmol/l Tris HCl, 0.01% (W/V) Tween 20, and 0.15 mol/l NaCl, pH 7.5 and the non-specifically-bound substance other than that of the antigen-antibody reaction was removed (BF separation).

Then, 200 μl of a ruthenium complex-labeled 3D6 antibody which had been diluted to a concentration of 4 μg/ml with a sandwich measurement reaction solution was added and reacted for 9 minutes at 30°C (second reaction). Subsequently, after trapping the magnetic beads with a magnet, the liquid was removed from the reaction cups and the magnetic beads were washed twice with 350 μl of a washing solution and the non-specifically-bound substance other than that of the antigen-antibody reaction was removed (BF separation).

After that, 300 μl of tryptophyl amine was introduced into each cup and mixed with the magnetic beads. The ruthenium complex emitted light when electrical energy was applied in this state and the luminescence intensity was detected with a detector. Moreover, the measuring procedure after the addition of the magnetic beads to the reaction cups described above was carried out with an automated ruthenium complex luminescence measurement device Picolumi 8220 (manufactured by Sanko Junyaku Co., Ltd.). A calibration curve was created using the measurement results of the standard Aβ1-42 synthetic peptide, and Aβ1-42 concentrations in the AD patients and the healthy people were calculated out using the calibration curve. FIG. 4 shows the results.

The results, as shown in FIG. 4, were such that, in 8 out of 25 cases with the AD patients and in 3 out of 25 cases with healthy people, the values were above 0.5 pg/ml which is the minimum sensitivity for measurement and in the cases with all of the remaining samples the values were below the measurement sensitivity. Further, it was confirmed that there was no clinical usefulness between the two groups of AD patients and healthy people according to a t-test in the case of the samples where measurement was possible ($p > 0.05$).

(Experiment Example 3) Fragmentation test of Aβ1-42 in serum

Serum from a healthy person (1 ml) was divided into 0.5 ml lots and 10 μl of a protease solution obtained by dissolving 1 protease inhibitor cocktail tablet (complete mini, manufactured by Roche Diagnostics K. K.) in 1 ml of a buffer solution containing 10 mmol/l Tris HCl (pH 7.5) was mixed with one of those lots (Experiment Example 3-1). Meanwhile, the other lot was mixed with 10 μl of a buffer solution containing 10 mmol/l Tris HCl (pH7.5) as a control (Experiment Example 3-2).

Then, 5 μl of a 1,000 ng/ml Aβ1-42 synthetic peptide (produced by Peptide Institute Inc.) was added to each serum. The serum to which the substances had been added was left to stand at 20°C and the change in the measured value of the added Aβ1-42 synthetic peptide with the passage of time was measured by the above-mentioned electrochemiluminescence double antibody sandwich measurement method as time progressed.

FIG. 5 is a graph which shows the percentage change of the measured value with the passage of time as a relative ratio taking the measured value immediately after the Aβ1-42 synthetic peptide had been added to the serum to be 100%. As shown in FIG. 5, the serum to which the Aβ1-42 synthetic peptide had been added after the addition of the protease inhibitor of Experiment Example 3-1 provided results that the reduction in the measured value with the passage of time had clearly been suppressed as compared with that of Experiment Example 3-2, that is, the control. This suggests that the Aβ1-42 in the plasma is rapidly fragmented by protease which is present in the blood.

FIG. 6 is a schematic drawing that conceptually shows metabolism of Aβ1-42 in blood. FIG. 6(a) shows the amino acid sequence (SEQ ID NO: 1) of Aβ1-42, and FIG. 6(b) shows the metabolism of Aβ1-42 in blood. Aβ1-42 is expressed in brain, and transferred into cerebrospinal fluid (CSF). After that, the Aβ1-42 leaks into blood, and then rapidly fragmented by proteases in the blood.

(Experiment Examples 4 and 5)

The enzyme degradation product of the Aβ1-42 synthetic peptide was measured by the electrochemiluminescence double antibody sandwich immunological assay (Experiment Example 4) and the competitive immunological assay using an immobilized antigen (Aβ1-42 synthetic peptide) (Experiment Example 5).

(a) Method of preparing respective samples

An enzyme-treated sample was prepared by: adjusting 20 μl of an Aβ1-42 synthetic peptide (produced by Peptide Institute Inc.) to have a concentration of 10 μg/ml with a 10 mmol/l potassium phosphate buffer solution (pH 7.8); and adding thereto 5 μl of trypsin (1:250 trypsin, produced by Sigma-Aldrich Corporation) which had been adjusted to have a concentration of 1 mg/ml with a 10 mmol/l potassium phosphate buffer solution (pH 7.8). In addition, a control sample was prepared in such a manner that 20 μl of an Aβ1-42 synthetic peptide (produced by Peptide Institute Inc.) having the same concentration as mentioned above was added with 5 μl of a 10 mmol/l potassium phosphate buffer solution (pH 7.8). Further, as a blank sample, a 10 mmol/l potassium phosphate buffer solution (pH 7.8) added with an Aβ1-42 synthetic peptide was used.

(b) Measurement of enzyme degradation product of Aβ1-42 synthetic peptide

The samples were each incubated for 30 minutes, and then subjected to measurement by the electrochemiluminescence double antibody sandwich immunological assay (Experiment Example 4) in the same manner as in Example 1 and a competitive immunological assay (Experiment Example 5) as described below.
Procedures of the competitive immunological assay of Experiment Example 5 are shown below.

(1) Method or preparing respective constitutional components of the reagent

(1-1) Method of preparing Aβ1-42 synthetic peptide-binding magnetic beads

0.3 ml of a 50 ng/ml Aβ1-42 synthetic peptide (produced by Peptide Institute Inc.) was mixed with 0.3 ml of 30 mg/ml magnetic beads (Dynabeads M-450 Epoxy, produced by Dynal Co.). The mixed liquid was stirred for 16 hours at 25°C and the synthetic peptide was bound to the magnetic beads. Subsequently, only the liquid solution was removed from the magnetic bead solution and the free synthetic peptide which was not bound to the magnetic beads remaining in the solution was removed. Then, 1 ml of phospholipid polymer reagent (S101E, produced by NOF Corporation) as a blocking agent was added and the mixture was stirred for 3 hours at 25°C. Subsequently, the magnetic beads were washed with 10 ml of the above-mentioned phospholipid polymer reagent (by washing 5 times with 2 ml of the phospholipid reagent). After washing, the Aβ1-42 synthetic peptide-binding magnetic beads were mixed with 0.3 ml of the above-mentioned phospholipid polymer reagent and stored at 4°C until they were to be used.

(1-2) Method of preparing a ruthenium complex-labeled 21F12 antibody

The 21F12 mouse monoclonal antibody (Innogenetics Inc.) which is an antibody specific to the C-terminal site of Aβ1-42 was diluted with a 10 mmol/l potassium phosphate buffer solution (pH 7.8) to an antibody concentration of 1 mg/ml. Then, 17.6 μl of a 10 mg/ml ruthenium complex (Origen TAG-NHS Ester, produced by Igen Co.) was added to 0.5 ml of the 1 mg/ml antibody, and the mixture was stirred for 30 minutes at 25°C. Subsequently, 30 μl of 2 mol/l glycine were added thereto, and the mixture was stirred for 30 minutes at 25°C.
Next, the ruthenium complex-labeled antibody solution was applied to gel filtration column chromatography (Sephadex G-25, manufactured by GE Healthcare Bio-Science KK) packed into a glass tube of diameter 1 cm and height 30 cm, and the ruthenium complex-labeled antibodies were isolated from the non-labeling ruthenium complex and purified. The elution was carried out with a 10 mmol/l potassium phosphate buffer solution (pH 6.0).

(2) Method of measuring Aβ x-42 in the sample

A plurality of reaction cups was prepared as required, and 50 μl of a reaction solution containing 50 mmol/l Tris HCl, 10% BSA, 0.15 mol/l NaCl, 0.01% (w/v) Tween 20, 10 mmol/l EDTA2Na, and 0.1% normal mouse serum (pH 7.5) was poured into each of the reaction cups. Each of the reaction cups was added with 50 μl of the enzyme-treated sample of the Aβ1-42 synthetic peptide and 50 μl of the control sample. The reaction cups were then added with 25 μl of an Aβ1-42 synthetic peptide-binding magnetic beads which had been diluted to have a concentration of 1.5 mg/ml with the reaction solution, and 200 μl of a ruthenium complex-labeled 21F12 mouse monoclonal antibody (hereinafter, referred to as "labeled antibody") which had been diluted to have a concentration of 0.1 μg/ml with the reaction solution. The mixtures were each stirred at 30°C for 26 minutes to allow a competitive reaction between the Aβ1-42 or the Aβ1-42 fragments in the samples and the magnetic beads-binding Aβ1-42 synthetic peptide, with the ruthenium complex-labeled 21F12 mouse monoclonal antibody to proceed.

Subsequently, after trapping the magnetic beads with a magnet, the liquid was removed from the reaction cups and the magnetic beads were washed twice with 350 μl of a washing solution containing 50 mmol/l Tris HCl, 0.01% (W/V) Tween 20, and 0.15 mol/l NaCl (pH 7.5) and the non-specifically-bound substance other than that of the antigen-antibody reaction was removed (BF separation). After that, 300 μl of tryptophyl amine was introduced into each cup and mixed with the beads. The ruthenium complex emitted light when electrical energy was applied in this state and the luminescence intensity was detected by a detector. In addition, the measuring procedure after the addition of the magnetic beads to the reaction cups described above was carried out with an automated ruthenium luminescence measurement device Picolumi 8220 (manufactured by Sanko Junyaku Co., Ltd.).

(c) Results

Table 2 shows the measurement results of the electrochemiluminescence double antibody sandwich immunological assay of Experiment Example 4. Table 3 shows the measurement results of the competitive immunological assay of Experiment Example 5. In Tables 2 and 3, the term "ECL value" indicates a measured value of intensity of the ruthenium complex luminescence, and the symbol "%" indicates a relative ratio of a measured value when a measured value of the control sample is regarded as 100%. In addition, in Table 3, the relative ratio of decrease in measurement value due to the competitive reaction when a value (blank value) of a sample without a competitive substance is regarded as 100% indicates an inhibition rate.

[Table 2]

| Measurement results of Experiment Example 4 (electrochemiluminescence double antibody sandwich measurement method) | | |
|---|---|---|
| Sample | ECL value | % |
| Blank | 692.5 | - |
| Control | 12794.8 | 100% |
| Enzyme treatment | 1092.3 | 9% |

[Table 3]

| Measurement results of Experiment Example 5 (competitive immunological assay) | | | |
|---|---|---|---|
| Sample | ECL value | Inhibition rate | % |
| Blank | 34375.8 | 0% | - |
| Control | 20842.7 | 39% | 100% |
| Enzyme treatment | 19560.6 | 43% | 94% |

As shown in Table 2, by the double antibody sandwich measurement method, from the trypsin treated Aβ1-42 synthetic peptide, it was only possible to detect 1/10 of the amount of Aβ1-42 detected in the control sample which had not been trypsin treated. However, as shown in Table 3, as a result of the measurement of the same samples with the competitive immunological assay, substantially the same competitive reaction was seen as that with the control sample which had not been treated with trypsin, confirming that even though the Aβ1-42 had been fragmented, the measurement can be performed in the same way as that for the full length Aβ1-42. That is, it was confirmed that the competitive immunological assay reflected accurately the amount of fragmented Aβ1-42 in the sample.

(Experiment Examples 6 and 7) 21F12 antibody characteristic tests

Aβ1-42 synthetic peptide (produced by Peptide Institute Inc.) (Experiment Example 6) and Aβ1-40 synthetic peptide (produced by Peptide Institute Inc.) (Experiment Example 7) which had been prepared at concentrations of 0 (blank), 10, 100, 1,000 and 10,000 ng/ml in a 10 mmol/l potassium phosphate buffer solution (pH 7.8) were measured using the competitive immunological assay. The competitive immunological assay was carried out using the procedure described in Example 5. FIG. 7 shows the results. Note that, the inhibition rate shown in the graph of FIG. 7 was represented by a relative ratio of the decrease in measured value obtained by the competitive reaction when a value (blank value) of a sample without a competitive substance was regarded as 100%.

The results, as shown in FIG. 7, were such that the competitive reaction increased as the concentration of the Aβ1-42 synthetic peptide of Experiment Example 6 increased while no competitive reaction occurred with the Aβ1-40 synthetic peptide of Experiment Example 7 even when the concentration increased. Therefore, it is clear that the antibody 21F12 used in the competitive immunological assay of the present invention is an antibody which reacts specifically with Aβ1-42 and which hardly reacts at all with Aβ1-40.

(Experiment Example 1) Measurement of AD patient and healthy person serum by the competitive immunological assay

The required number of cups into which 50 μl of the reaction solution had been introduced was used. Then, 50 μl of serum from each of 25 AD patients, serum from each of 15 healthy people, and a reaction solution (blank) were mixed respectively as samples. Then, 25 μl of Aβ1-42 synthetic peptide-binding magnetic beads diluted to a concentration of 1.5 mg/ml with the reaction solution was added and measurements were made by the competitive immunological assay in accordance with the procedure described in Example 5. FIG. 8 shows the results.

The measured results showed that the luminescence intensity for each sample obtained was reduced when compared with the luminescence intensity in the case where reaction solution had been used as a sample which contained no competitive material, since when there is a large amount of Aβx-42 in the sample, the amount of the ruthenium complex-labeled antibody bound to the magnetic bead-bound peptide is reduced by the competitive reaction.
The luminescence intensity obtained was calculated as an inhibition factor using the following equation:

Inhibition Factor (%) = [1 - Luminescence intensity of each sample/Luminescence intensity when reaction solution is used as the sample (luminescence intensity when there is no competitive reaction)] x 100.

The results, as shown in FIG. 8, were assessed as being significantly different with a probability of more than 95% between the two groups of AD patients and healthy people in a t-test ($p < 0.0002$).

(Example 2) Competitive immunological assay using a polyclonal antibody

Measurement of serum samples of 20 AD patients and 18 healthy people was performed by the competitive immunological assay in the same manner as in Example 1 except that, in stead of the ruthenium complex-labeled 21F12 antibody, a ruthenium complex-labeled AB5078P antibody which was obtained by labeling a polyclonal antibody AB5078P (produced by Chemicon International, Inc.) which specifically reacts with the C-terminal site of Aβ1-42 with a ruthenium complex. FIG. 9 shows the results.
As a result, as in Example 1, it was determined that there was a significant difference between two groups, that is, a group of the AD patients and a group of the healthy people, by the t-test with an probability of 95% or higher ($p < 0.0002$). The result revealed that any one of the monoclonal antibody and the polyclonal antibody can be used as long as the antibody is specific to the C-terminal site of Aβ1-42.

(Example 3) Competitive immunological assay using an immobilized antibody

(a) Method of preparing respective constitutional components of the reagent

(1) Method of preparing Aβ1-42 C-terminal site-retaining peptide labeled with a ruthenium complex

(1-1) Preparation of thiolated SPDP-binding ruthenium complex-labeled polylysin

Polylysine (poly-L-lysine, average molecular weight of 38,000, produced by Sigma-Aldrich Corporation) (2.5 ml) was dissolved in 2 ml of phosphate buffer (pH 7.8), then 450 μl of a 10 mg/ml ruthenium complex (Origen TAG-NHS Ester, produced by Igen Co.) was added and mixed for 60 minutes at 25°C. Then, 200 μl of 2 mol/l glycine was added to the mixture and mixed for 30 minutes at 25°C. The mixture was applied to gel filtration column chromatography (Sephadex G-25, manufactured by GE Healthcare Bio-Science KK) packed into a glass tube of internal diameter 2 cm and height 40 cm and the ruthenium complex-labeled polylysine was isolated from the non-labeling ruthenium complex and purified. The elution was carried out with a 10 mmol/l potassium phosphate buffer solution (pH 6.0).

Then, 8.3 μl of a 20 mmol/l protein crosslinking agent (SPDP, produced by Pierce Biotechnology, Inc.) was mixed with the 2 mg/ml ruthenium complex-labeled polylysine and the mixture was stirred for 1 hour at 25°C while blowing in nitrogen gas. After stirring, the mixture was applied to gel filtration column chromatography (Sephadex G-25, manufactured by GE Healthcare Bio-Science KK) packed into a glass tube of internal diameter 2 cm and height 40 cm and the SPDP-bound ruthenium complex-labeled polylysine was isolated from the unbound SPDP and refined. The elution was carried out with a 10 mmol/l potassium phosphate buffer solution which contained 1 mmol/l of ethylenediamine tetra-acetic acid di-sodium salt (pH 7.5).

Then, 55 μl of a 10 mmol/l potassium phosphate buffer solution which contained 1 mol/l dithiothreitol and 1 mmol/l ethylenediamine tetra-acetic acid di-sodium salt were added to the SPDP-bound ruthenium complex-labeled polylysine and the 2-pyridyl di-sulfide groups of the SPDP were thiolated by stirring the mixture for 30 minutes at 25°C while blowing in nitrogen gas. After stirring, the mixture was applied to gel filtration column chromatography (Bio-Gel P-4, manufactured by Bio-Rad laboratories Inc.) packed into a glass tube of internal diameter 1 cm and height 30 cm and the thiolated SPDP-bound ruthenium complex-labeled polylysine was isolated from the dithiothreitol and thiolated by-products and purified. The elution was carried out with a 10 mmol/l potassium phosphate buffer solution which contained 1 mmol/l of ethylenediamine tetra-acetic acid di-sodium salt (pH 7.5).

(1-2) Preparation of SPDP-bound 15L-β33-42 peptide

0.5 mg of an Aβ33-42 synthetic peptide in a form with 15 lysine residues bound on the N-terminal side, having an amino acid sequence: KKKKKKKKKKKKKKKGLMVGGVVIA (SEQ ID NO: 2), hereinafter, referred to as 15L-β33-42 was dissolved in 50 μl of DMSO and diluted with 0.45 ml of a 10 mmol/l potassium phosphate buffer solution which contained 1 mmol/l ethylenediamine tetra-acetic acid di-sodium salt (pH 7.5) to prepare a 15L-β33-42 peptide solution. Then, 50 μl of a 20 mmol/l protein crosslinking agent (SPDP, produced by Pierce Biotechnology, Inc.) was mixed with the 15L-β33-42 peptide solution, and the mixture was stirred for 1 hour at 25°C while blowing in nitrogen gas. After stirring, the mixture was applied to gel filtration column chromatography (Bio-Gel P-4, manufactured by Bio-Rad laboratories Inc.) packed into a glass tube of internal diameter 1 cm and height 30 cm and the SPDP-bound 15L-β33-42 peptide was isolated from the unbound SPDP and purified. The elution was carried out with a 10 mmol/l potassium phosphate buffer solution which contained 1 mmol/l ethylenediamine tetra-acetic acid di-sodium salt (pH 7.5).

(1-3) Binding of thiolated SPDP-bound ruthenium complex-labeled polylysine with SPDP-bound 15L-β33-42 peptide

0.3 mg of thiolated SPDP-bound ruthenium complex-labeled polylysine was added to 0.2 mg of SPDP-bound 15L-β33-42 peptide and left to stand for 16 hours at 4°C, and the 15L-β33-42 peptide and the ruthenium complex-labeled polylysine were bound via 2-pyridyl di-sulfide binds of the 15L-β33-42 peptide-bound SPDP and the thiol groups of the thiolated SPDP-bound ruthenium complex-labeled polylysine. The mixture was applied to gel filtration column chromatography (Sephadex G-25, manufactured by GE Healthcare Bio-Science KK) packed into a glass tube of internal diameter 1 cm and height 30 cm and the 15L-β33-42 peptide + ruthenium complex-labeled polylysine-bound substance (hereinafter, referred to as "labeled peptide composite") was isolated from the free SPDP-bound 15L-β33-42 peptide and the free thiolated SPDP-bound ruthenium complex-labeled polylysine and purified. The elution was carried out with a 10 mmol/l potassium phosphate buffer solution which contained 1 mmol/l ethylenediamine tetra-acetic acid di-sodium salt (pH 7.5). Note that, the bound substances of the peptide and the ruthenium complex-labeled polylysine can be produced by means of the same method using other peptide fragments which retain the Aβ1-42 C-terminal site and Aβ1-42 synthetic peptide instead of the 15L-β33-42 peptide used above.

(2) Method of preparing magnetic beads bound with an antibody specific to C-terminal site of Aβ1-42

21F12 antibody-binding magnetic beads were prepared in the same manner as in Experiment Example 1. Note that, other antibodies can be used instead of the 21F12 antibody as long as they are specific to the C-terminal site of Aβ1-42.

(b) Measurement of serum from AD patients and healthy people

A plurality of 500-μl polystyrene cups (hereinafter, referred to as "reaction cups") were prepared as required,

and 50 μl of a reaction solution containing 10 mmol/l sodium phosphate, 1% Block Ace, 0.15 mol/l NaCl, 0.01% (W/V) Tween 20, 10 mmol/l EDTA2Na, 0.25% (W/V) trehalose, and 0.1% normal mouse serum (pH 7.2) was poured into each of the reaction cups. 20 μl aliquots of an Aβ1-42 synthetic peptide as standards for calibration curve purposes at optional concentrations of 0, 31.25, 62.5, 125, 250, 500, and 1,000 ng/ml were mixed in the reaction cups, respectively, and 20 μl aliquots of serum from 15 AD patients and 15 healthy people as samples for measurement were mixed in the reaction cups. Then, 25 μl aliquots of 21F12 antibody-binding magnetic beads which had been diluted to a concentration of 0.3 mg/ml with reaction solution were added and 200 μl of a labeled peptide composite diluted to a concentration of 1 μg/ml with reaction solution were added and reacted for 26 minutes at 30°C.

Subsequently, the liquid in the reaction cups was removed while trapping the magnetic beads with a magnet and the magnetic beads were washed twice with 350 μl of a washing solution containing 50 mmol/l Tris HCl, 0.1% (W/V) Tween 20, and 0.15 mol/l NaCl (pH 7.5) and the non-specifically-bound substance was removed.
Subsequently, 300 μl of tryptophyl amine was introduced into each reaction cup and mixed with the magnetic beads. The ruthenium complex emitted light on applying electrical energy in this state and the luminescent intensity was detected with detection apparatus. Moreover, the procedure following the addition of the magnetic beads to the reaction cups described above was carried out using an automated ruthenium luminescence measurement device Picolumi 8220 (manufactured by Sanko Junyaku Co., Ltd.).

The inhibition rates due to the competitive reaction with the standard Aβ1-42 synthetic peptide are shown in Table 4 and a calibration curve (y = -3107.3, Ln(x) + 26,227, $R^2$ = 0.9909) is shown in FIG. 10. The measured values for the AD patient and healthy person serum samples are shown in Tables 5 and 6 and in FIG. 11. Note that the determined values were calculated by applying ECL values of the samples to the calibration curve of FIG. 10. The results confirmed that the total amount of Aβ1-42 C-terminal site in the serum is significantly lower in AD patients than in healthy people and that the means of the present invention can be used for the diagnosis of AD.

[Table 4]

| Measurement results of standard product of Experiment Example 3 | | |
|---|---|---|
| Aβ1-42 synthetic peptide (ng/mL) | ECL value | Inhibition rate (%) |
| 1000 | 4912.7 | 71 |
| 500 | 6523.8 | 62 |
| 250 | 9385.7 | 45 |
| 125 | 11437.7 | 33 |
| 50 | 13427.0 | 21 |
| 25 | 16583.3 | 3 |
| 0 (Blank) | 17024.9 | 0 |
| * The inhibition rate in the table is, where the luminescence intensity measured value (ECL value) of the ruthenium complex with no inhibition (concentration of added Aβ1-42 peptide is 0, that is, blank) was taken to be 100%, a reduction rate of the ECL luminescence intensity when each concentration of Aβ1-42 was introduced as a percentage. | | |

[Table 5]

| Measurement results of AD patient's serum of Example 3 | | | |
|---|---|---|---|
| Sample No. | ECL value | Determined value (ng/mL) | Average of the determined values (ng/mL) |
| 1 | 8177.0 | 333.1 | |
| 2 | 10132.0 | 177.6 | |
| 3 | 8510.2 | 299.2 | |
| 4 | 16509.6 | 22.8 | |

(continued)

| Measurement results of AD patient's serum of Example 3 | | | |
|---|---|---|---|
| Sample No. | ECL value | Determined value (ng/mL) | Average of the determined values (ng/mL) |
| 5 | 14690.0 | 41.0 | 107 |
| 6 | 15315.1 | 33.5 | |
| 7 | 16859.0 | 20.4 | |
| 8 | 11183.0 | 126.6 | |
| 9 | 13187.3 | 66.4 | |
| 10 | 13964.6 | 51.7 | |
| 11 | 12901.8 | 72.8 | |
| 12 | 14652.2 | 41.5 | |
| 13 | 10213.5 | 173.0 | |
| 14 | 13876.8 | 53.2 | |
| 15 | 12331.5 | 87.5 | |

[Table 6]

| Measurement results of healthy person's serum of Example 3 | | | |
|---|---|---|---|
| Sample No. | ECL value | Determined value (ng/mL) | Average of the determined values (ng/mL) |
| 1 | 6373.8 | 595.1 | 570 |
| 2 | 9165.9 | 242.3 | |
| 3 | 7037.7 | 480.6 | |
| 4 | 6793.5 | 519.9 | |
| 5 | 6031.1 | 664.5 | |
| 6 | 7804.0 | 375.6 | |
| 7 | 5717.6 | 735.0 | |
| 8 | 6681.6 | 539.0 | |
| 9 | 5920.4 | 688.6 | |
| 10 | 6433.4 | 583.8 | |
| 11 | 6152.4 | 639.1 | |
| 12 | 5036.9 | 915.0 | |
| 13 | 7445.8 | 421.5 | |
| 14 | 6910.4 | 500.7 | |
| 15 | 6138.2 | 642.0 | |

Note that, the calibration curve with this procedure was drawn up using Aβ33-42 synthetic peptide as the standard antigen but it was confirmed that the same results as those shown in FIG. 10 were observed with a calibration curve drawn up using Aβ1-42 synthetic peptide as the standard antigen. Further, the antibody used was the same 21F12 as used in Example 1 and it was confirmed that a competitive reaction was seen with Aβ1-42 synthetic peptide and Aβ33-42 synthetic peptide but that there was no reaction with Aβ1-40 synthetic peptide.

(Experiment Example 8) Test for confirming Aβ1-42 fragmentation in serum

Investigation was performed on change in molecule thereof after the Aβ1-42 synthetic peptide had been added to serum by SDS-PAGE electrophoresis.

1. Preparation of samples

In Experiment Example 8-1, 50 μl of a serum sample from a healthy person was mixed with 500 μl of an Aβ1-42 synthetic peptide (produced by Peptide Institute Inc.) of a concentration of 50 μg/ml, and the mixture was well stirred, followed by incubation at 25°C for 90 hours. The resultant sample was subjected to purification, desalination, and concentration by the following method to prepare a purified desalted sample (30 μl).
In Experiment Example 8-2, 50 μl of a serum sample from a healthy person was mixed with 500 μl of the Aβ1-42 synthetic peptide (produced by Peptide Institute Inc.) of a concentration of 50 μg/ml, and the mixture was well stirred. The resultant sample was immediately subjected to purification, desalination, and concentration by the following method to prepare a purified desalted sample (30 μl).
In Experiment Example 8-3, to confirm the molecular weight of Aβ in serum of a living body, 1 ml of pool serum from a healthy person was subjected to purification, desalination, and concentration by the following method to prepare a purified desalted sample (30 μl).
The method for the purification, desalination, and concentration of the samples was as described below. A sample was purified using affinity gel in which a 21F12 antibody (produced by Innogenetics Inc.) that recognizes the C-terminal of Aβ1-42 was bound to sepharose gel (CNBr-activated sepharose4B: produced by GE Healthcare Bio-Science KK). The purification was performed in such a manner that: 0.2 ml of the 21F12 antibody affinity gel was packed into an Ultrafree-MC (manufactured by Millipore Corporation), and then a sample to which the Aβ1-42 synthetic peptide had been added was applied thereto. After that, 10 mM sodium phosphate/0.15 M NaCl (pH 7.8) was allowed to flow through the column for washing until a discharged solution flew out from the column had the absorbance at A280 nm of 0.000. After that, 0.1 M citric acid (pH 5.0) was allowed to flow through the column for washing until a discharged solution flew out from the column had the absorbance at A280 nm of 0.000. The Aβ which had been bound to the 21F12 column was eluted out with 0.6 ml of 4 M guanidine, and the resultant eluate was subjected to desalination and concentration using Zip-Tip C18 (manufactured by Millipore Corporation) to yield 30 μl of a purified sample.

2. Measurement by competitive immunological assay

The samples obtained by the desalination and concentration were each subjected to measurement by the competitive immunological assay as described in Example 3. The measurement was performed in such a manner that: 2 μl each of the purified samples was diluted 10-fold with a reaction solution; and 20 μl of the dilution was used as a sample. Table 8 shows the measurement results for the purified desalted samples. In addition, Table 7 shows the measurement results for the standard Aβ1-42 synthetic peptide. FIG. 12 shows a calibration curve (y = -3545.7, Ln(x) + 30507, $R^2$ = 0.9856).
As a result, a determined value in Experiment Example 8-1 was 452 ng/ml. Since the purified sample of this case had been diluted 10-fold in advance, the purified sample had an Aβx-42 concentration of 4,520 ng/ml, and the amount of Aβx-42 in the sample was calculated to be 136 ng because the purified sample had a volume of 30 μl.
[Table 7]

| Measurement results of standard product of Experiment Example 8 | |
|---|---|
| Aβ1-42 Synthetic peptide (ng/mL) | ECL value |
| 1000 | 6270.0 |
| 500 | 8411.9 |
| 250 | 10226.7 |
| 125 | 13950.9 |
| 62.5 | 15788.9 |
| 0 (Blank) | 18133.2 |

[Table 8]

| | ECL value | Determined value (ng/ml) | Value after concentration conversion (10-fold) | Yield (ng) |
|---|---|---|---|---|
| Experiment Example 8-1 | 8829.1 | 452 | 4521 | 136 |
| Experiment Example 8-2 | 7589.1 | 641 | 6414 | 192 |
| Experiment Example 8-3 | 11305.6 | 225 | 2248 | 67 |

Experiment Example 8-1: sample obtained by adding an Aβ1-42 synthetic peptide to serum and purifying the mixture after 90 hours from the addition
Experiment Example 8-2: sample obtained by adding an Aβ1-42 synthetic peptide to serum and purifying the mixture immediately after the addition
Experiment Example 8-3: sample obtained by purifying 1 ml of serum

3. Electrophoresis

Next, the purified desalted samples were subjected to electrophoresis and emerged bands were observed. Nu-PAGE Bis-Tris gel (produced by Invitrogen Corporation) having a 4-12% gradient was used as electrophoresis gel, and Mark 12 (produced by Invitrogen Corporation) was used as a molecular marker. The purified desalted samples were each mixed with an LDS sample buffer (produced by Invitrogen Corporation) that was a sample dilution buffer so that 50 ng each of the samples were applied to the gel, according to the determined values obtained by the competitive immunological assay, and the mixtures were each applied to the gel. At the same time, optional amounts of the Aβ1-42 synthetic peptide (produced by Peptide Institute Inc.) and an Aβ34-42 synthetic peptide (produced by Sigma-Aldrich Corporation) were each mixed with the LDS sample buffer, and each of the mixtures and 5 μl of the molecular marker were applied to the gel. Electrophoresis was performed at 15 mA until a blue pigment in the LDS sample buffer reached the bottom of the gel. After the electrophoresis, the gel was subjected to silver staining using a silver staining II kit Wako (manufactured by Wako Pure Chemical Industries, Ltd.) to stain protein bands. FIG. 13 shows a photograph of the stained gel after the electrophoresis.

As a result of the electrophoresis, the Aβ1-42 synthetic peptide exhibited a band at around a molecular weight of 4.5 KDa by referring to the result of the molecular marker. On the other hand, the Aβ34-42 synthetic peptide that had a molecular weight of 0.8581 KDa did not exhibit a band. The results of the electrophoresis of the molecular marker indicated that peptides having molecular weights of 3.5 KDa or more was able to be detected, but the Aβ34-42 having additionally smaller molecular weight had migrated to the bottom of the gel, so the Aβ34-42 was not able to be detected as a band on the gel.
For the sample obtained by adding Aβ1-42 to serum and purifying the mixture immediately after the addition by using the 21F12 antibody affinity column (Experiment Example 8-2), a band was detected at the same position as that of the Aβ1-42 synthetic peptide. In addition, there was no band detected in molecular weight regions except that at around 4.5 KDa. Thus, it was confirmed that, by using the present purification method using the 21F12 antibody affinity column, the Aβ1-42 which had been added to the serum was able to be purified from the serum to a state where the Aβ1-42 alone exists.

On the other hand, for the sample which had been purified using the 21F12 affinity column after the addition and incubation for 90 hours (Experiment Example 8-1) and the sample obtained by purifying 1 ml of serum (Experiment Example 8-3), there was no band at around 4.5 KDa, and there was no band detected in molecular weight regions except that at around 4.5 KDa. In those cases, 50 ng of each of the samples was applied to the gel according to the determined values obtained by the competitive immunological assay. Thus, after the addition of the Aβ1-42 synthetic peptide to the serum, the synthetic peptide in the serum was gradually fragmented with passage of time and converted into small molecules, so it was considered that the band of the Aβ1-42 synthetic peptide was not able to be detected for the same reason as that for the Aβ34-42 synthetic peptide.

(Experiment Example 9)

For a method of confirming the presence or absence of Aβx-42 fragments in molecular weight regions of 3.5 KDa or less, which cannot be detected by SDS-PAGE, an investigation was made on a method using a low-molecular peptide separation gel filtration column.
Biogel P-4 gel (produced by Bio-Rad laboratories Inc.) that was a gel filtration column was packed into a glass column tube (manufactured by Bio-Rad laboratories Inc.) having φ of 1.0 and height of 120 cm so that a gel volume was 84 ml. A mobile phase of 8M urea/50 mM Tris (pH 7.5) was sent at a flow speed of 0.1 ml/min with an HPLC pump CCPM (manufactured by Tosoh Corporation). An eluate from the column was measured for absorption thereof at A225 nm with an SPD6AV spectrometer (manufactured by Shimadzu Corporation), and measurement data was used for creation of a chart with a C-R3A chromatopack (manufactured by Shimadzu Corporation). Note that, the SPD-6AV spectrometer was set to have a range of 0.005 and a response of STD mode. The C-R3A chromatopack was set to have peak detection sensitivity (SLOPE) of 193.68, a minimum peak area value of 10, and a chart speed of 1 mm/min.

The gel filtration column apparatus was applied with pepsin A (molecular weight of 35 KDa, produced by Sigma-Aldrich Corporation), Aβ1-42 (molecular weight of 4.514 KDa, produced by Peptide Institute Inc.), Aβ1-28 (molecular weight of 3.2625 KDa, produced by Bachem AG), Aβ12-28 (molecular weight of 1.9552 KDa, produced by Sigma-Aldrich Corporation), Aβ1-11 (molecular weight of 1.3253KDa, produced by Bachem AG), Aβ34-42 (molecular weight of 0.8581 KDa, produced by Sigma-Aldrich Corporation), Suc-D-Asp-MCA (molecular weight of 0.3903 KDa, produced by Peptide Institute Inc.), and E-64-c (molecular weight of 0.31438 KDa, produced by Peptide Institute Inc.) having dissolved in dimethylsulfoxide (hereinafter, referred to as "DMSO", produced by Sigma-Aldrich Corporation), and DMSO alone (molecular weight of 0.0781 KDa, produced by Sigma-Aldrich Corporation) in this order. Charts were created with a chromatopack using absorption at A225 nm of eluates thereof. FIG. 14 shows the charts. In addition, FIG. 15 shows a graph created using the peak emergence time shown in the charts and the known molecular weights.

Due to the linearity between the pepsin A (molecular weight of 35 KDa) and Aβ1-42 (molecular weight of 4.51 KDa) shown in the graph of FIG. 15, the pepsin A and Aβ1-42 were considered to be eluted at exclusion limit values (Void volume) of the column. Therefore, fractionation molecular weights used in the gel filtration column apparatus were 3.2625 KDa (Aβ1-28) to 0.0781 KDa (DMSO), and a calibration curve within this molecular weight range was created. FIG 16 shows a graph of the thus-created calibration curve (y = -3545.7, Ln(x) + 30507, $R^2$ = 0.9856).
In addition, to investigate reproducibility of the apparatus, Aβ1-28, Aβ1-11, and Suc-D-Asp-MCA were again applied to the column to confirm differences in elution time between the first application and the second application. As a result, good reproducibility was attained. FIG. 17 shows a chart, and Table 9 shows reproducibility data.

[Table 9]

| Results of Experiment Example 9 | | | |
|---|---|---|---|
| Sample | Elution time (min) | | Relative ratio (%) |
| | First | Second | |
| Aβ1-28 | 287.622 | 286.467 | 99.60 |
| Aβ1-11 | 342.513 | 343.367 | 100.25 |
| Suc-D-Asp-MCA | 494.522 | 492.400 | 99.57 |

In Table 9, the relative ratio is represented as % of a second measured value regarding a first value as 100.

(Experiment Example 10)

To confirm the molecular weight of Aβ in serum of a living body, 1 ml of pool serum from a healthy person was subjected to purification using affinity gel in which a 21F12 antibody which recognizes the C-terminal of Aβ1-42 was bound to sepharose gel (CNBr-activated sepharose4B). The purification was performed in such a manner that: 0.2 ml of the affinity gel was packed into an Ultrafree-MC (manufactured by Millipore Corporation), and then a sample was applied thereto, and a passed fraction was preserved at -20°C. After that, 10 mM sodium phosphate/0.15 M NaCl (pH 7.8) was allowed to flow through the column for washing until a discharged solution flew out from the column had the absorbance at A280 nm of 0.000. After that, 0.1 M citric acid (pH 5.0) was allowed to flow through the column for washing until a discharged solution flew out from the column had the absorbance at A280 nm of 0.000. A 21F12 column-bound fraction was eluted out with 0.4 ml of 8M urea/50 mM Tris (pH 7.5).
A purified sample was added with 5 μl of DMSO, and then applied to the gel filtration column apparatus, and a chart was created with a chromatopack by using absorption at A225 nm of the eluate. FIG. 18 shows the chart.

DMSO which had been added to the sample exhibited a peak thereof at 678.8 minutes, which resulted in good reproducibility as compared with the peak emergence time of DMSO at the time of creation of the calibration curve of 676.567 minutes. The resultant sample had a main peak at 512.167 minutes. There was a shoulder peak prior to the main peak, which was not sufficiently separated from the main peak, and the emergence time of the shoulder peak was about 470 minutes when converted from a chart speed and a distance from the beginning to the shoulder peak. Although not able to be visually observed, a plurality of extremely small detected peaks were detected, which were able to be detected by an automated peak detection mechanism of the chromatopack. The emergence time of the main peak and the shoulder peak was applied to the previously-created calibration curve of FIG. 19 to calculate out molecular weights of the eluted substances. As a result, the main peak had a molecular weight of 0.357 KDa and the shoulder peak had a molecular weight of 0.526 KDa.

In addition, the passed fraction collected from the affinity column and pool serum from a healthy person (same as that purified) which had not been purified were subjected to measurement by the competitive immunological assay as described in Example 3. As a result, the serum before being subjected to affinity purification had a measured value of 875 ng/ml while the passed fraction which was obtained by allowing the serum to pass through the 21F12 column had a measured value of 293 ng/ml, resulting in an decrease in amount to about 30% of that before passage through the column. Therefore, the balance, that is, about 70% (582 ng), of a total amount was considered to be bound to the 21F12 affinity column, and the low-molecular peak emerged in the chart obtained by using the gel filtration column apparatus reflected the amount of about 70% (582 ng) which corresponded to the decrease in value obtained by the competitive immunological assay.

Those results revealed that an Aβx-42 fragment at the main peak was estimated to have a C-terminal peptide of Val-Ile-Ala (molecular weight of 0.33742 KDa), and an Aβx-42 fragment at the shoulder peak was estimated to have a C-terminal peptide of Gly-Val-Val-Ile-Ala (0.52964 KDa).

In addition, since the plurality of extremely small peaks were detected in the automated peak detection of the chromatopack, there was a possibility that a plurality of Aβx-42 fragments, other than those at the main peak and the shoulder peak, were present in the sample which had been obtained by purifying serum with the 21F12 antibody affinity column.

The results of the investigation using SDS-PAGE of Experiment Example 8, the investigations using the gel filtration column apparatus of Experiment Examples 9 and 10, and the metabolism confirmation test using the protease inhibitor of Experiment Example 3 strongly supported the idea that Aβ1-42 is degraded by enzymes and fragmented in blood. This finding demonstrated for the first time that the Aβx-42 fragments were able to be measured using a blood sample such as serum or plasma. Accordingly, the finding means that the present invention is effective for every method of measuring an Aβ fragment by using a blood sample such as serum or plasma as well as for the competitive immunological assay disclosed by the present invention.

Industrial Applicability

Measurement of the total amount of Aβ1-42 and peptide fragments which retain the Aβ1-42 C-terminal site of Aβ1-42 (Aβx-42) in a sample using an immunological assay with antibodies which are specific to the C-terminal site of Aβ1-42 can be used for the diagnosis of Alzheimer's disease.

## Claims

1. A method of assaying Alzheimer's disease, comprising measuring a total amount of β-amyloid 1-42 and β-amyloid 1-42 fragments each of which retains a C-terminal site of the β-amyloid 1-42 in a biological sample by an immunological assay in which an antibody specific to the C-terminal site of the β-amyloid 1-42 is used.

2. The method of assaying Alzheimer's disease according to claim 1, wherein the immunological assay is a competitive immunological assay.

3. The method of assaying Alzheimer's disease according to claim 1 or 2, wherein the antibody is a polyclonal antibody or a monoclonal antibody.

4. The method of assaying Alzheimer's disease according to claim 3, wherein the monoclonal antibody is an antibody which recognizes β-amyloid 1-42 but which does not recognize β-amyloid 1-40.

5. The method of assaying Alzheimer's disease according to any one of claims 1 to 4, wherein the antibody is a mouse monoclonal antibody specific to a C-terminal site of Aβ1-42, which is obtained by using as an immunogen a 33-42 amino acid site of the β-amyloid 1-42.

6. The method of assaying Alzheimer's disease according to claim 2, wherein the competitive immunological assay comprises the following steps (a) and (b) of:

(a) reacting a support on which a peptide which retains the C-terminal site of the β-amyloid 1-42 is immobilized as a solid phase, a biological sample, and an antibody which recognizes the C-terminal site of the β-amyloid 1-42 and which is labeled with a labeling substance; and
(b) measuring, after washing, a total amount of the β-amyloid 1-42 and β-amyloid 1-42 fragments each of which retains the C-terminal site of the β-amyloid 1-42 by measuring an amount of a labeled antibody which is bound to the peptide which retains the C-terminal site of the β-amyloid 1-42 and which is immobilized as a solid phase.

7. The method of assaying Alzheimer's disease according to claim 2, wherein the competitive immunological assay comprises the following steps (a) and (b) of:

(a) reacting a support on which an antibody which recognizes the C-terminal site of the β-amyloid 1-42 is immobilized as a solid phase, a biological sample, and a peptide which retains the C-terminal site of the β-amyloid 1-42 and which is labeled with a labeling substance; and
(b) measuring, after washing, a total amount of β-amyloid 1-42 and β-amyloid 1-42 fragments each of which retains the C-terminal site of the β-amyloid 1-42 by measuring an amount of the labeled peptide which retains the C-terminal site of the β-amyloid 1-42 and which is bound to the immobilized antibody.

8. The method of assaying Alzheimer's disease according to claim 7, wherein the peptide which retains the C-terminal site of β-amyloid 1-42 and which is labeled with the labeling substance is a peptide composite which has a carrier substance bound thereon.

9. The method of assaying Alzheimer's disease according to claim 8, wherein the carrier substance comprises polylysine, dextran, bovine serum albumin, or a polypeptide which has many free amino groups.

10. The method of assaying Alzheimer's disease according to any one of claims 6 to 9, wherein the labeling substance comprises a ruthenium complex.

11. The method of assaying Alzheimer's disease according to any one of claims 6 to 10, wherein the support comprises magnetic beads.

12. The method of assaying Alzheimer's disease according to any one of claims 1 to 11, wherein the biological sample comprises serum or plasma.

13. A diagnostic reagent for Alzheimer's disease, comprising as an essential constitutional component an antibody specific to a C-terminal site of β-amyloid 1-42, wherein the diagnostic reagent is used for assaying Alzheimer's disease by measuring a total amount of the β-amyloid 1-42 and β-amyloid 1-42 fragments each of which retains the C-terminal site of the β-amyloid 1-42 in a biological sample.

14. The diagnostic reagent for Alzheimer's disease according to claim 13, further comprising as essential constitutional components:

a peptide which retains the C-terminal site of the β-amyloid 1-42 and which is immobilized as a solid phase; and an antibody which is specific to the C-terminal site of the β-amyloid 1-42 and which is labeled with a labeling substance,

wherein the diagnostic reagent is used for a competitive immunological assay.

**15.** The diagnostic reagent for Alzheimer's disease according to claim 13, further comprising as essential constitutional components:

an antibody which is specific to a C-terminal site of β-amyloid 1-42 and which is immobilized as a solid phase; and a peptide which retains the C-terminal site of the β-amyloid 1-42 and which is labeled with a labeling substance,

wherein the diagnostic reagent is used for a competitive immunological assay.

**16.** The diagnostic reagent for Alzheimer's disease according to claim 14 or 15, wherein the support to be used for immobilization comprises magnetic beads.

**17.** The diagnostic reagent for Alzheimer's disease according to claim 15, wherein the peptide which retains the C-terminal site of the β-amyloid 1-42 which is labeled with the labeling substance comprises a peptide composite having a carrier substance bound thereto.

**18.** The diagnostic reagent for Alzheimer's disease according to claim 17, wherein the carrier substance comprises polylysine, dextran, bovine serum albumin, or a polypeptide which has many free amino groups.

**19.** The diagnostic reagent for Alzheimer's disease according to any one of claims 14 to 17, wherein the labeling substance comprises a ruthenium complex.

**20.** The diagnostic reagent for Alzheimer's disease according to any one of claims 13 to 19, wherein the antibody comprises a polyclonal antibody or a monoclonal antibody.

**21.** The diagnostic reagent for Alzheimer's disease according to claim 20, wherein the monoclonal antibody comprises an antibody which recognizes the β-amyloid 1-42 but which does not recognize β-amyloid 1-40.

**22.** The diagnostic reagent for Alzheimer's disease according to any one of claims 13 to 21, wherein the antibody comprises a mouse monoclonal antibody which is specific to a C-terminal site of Aβ1-42 and which is obtained by using as an immunogen a 33-42 amino acid site of the β-amyloid 1-42.

**23.** The diagnostic reagent for Alzheimer's disease according to any one of claims 13 to 22, wherein the biological sample comprises serum or plasma.

**24.** A method of measuring β-amyloid 1-42, comprising measuring an amount of β-amyloid 1-42 in a biological sample by measuring a total amount of the β-amyloid 1-42 and β-amyloid 1-42 fragments each of which retains the C-terminal site of the β-amyloid 1-42 in a biological sample by an immuno assay method in which an antibody specific to the C-terminal site of the β-amyloid 1-42 is used.

**25.** The method of measuring β-amyloid 1-42 according to claim 24, wherein the biological sample comprises serum or plasma.

FIG.1

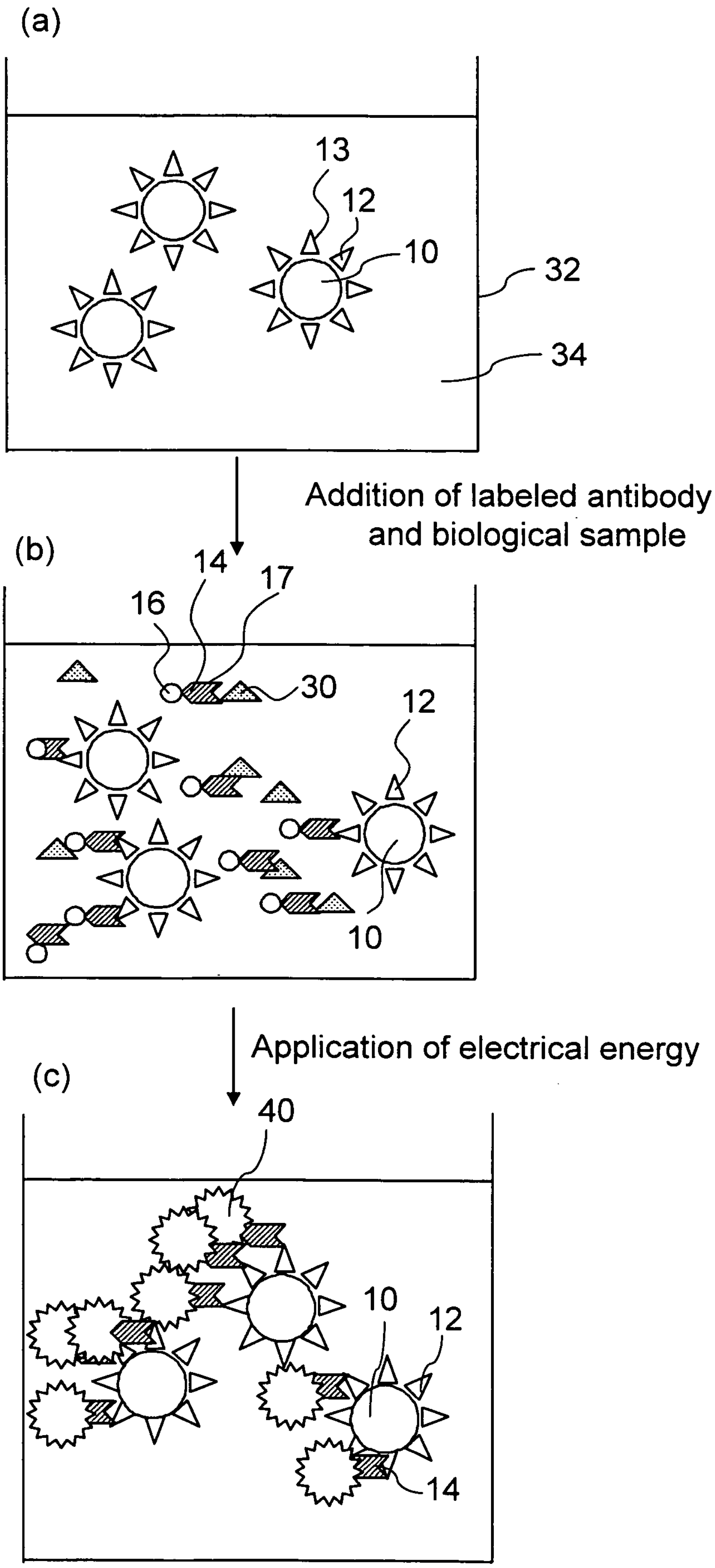
(a)
13
12
10
32
34
Addition of labeled antibody and biological sample
(b)
14
17
16
30
12
10
Application of electrical energy
(c)
40
10
12
14

FIG.2

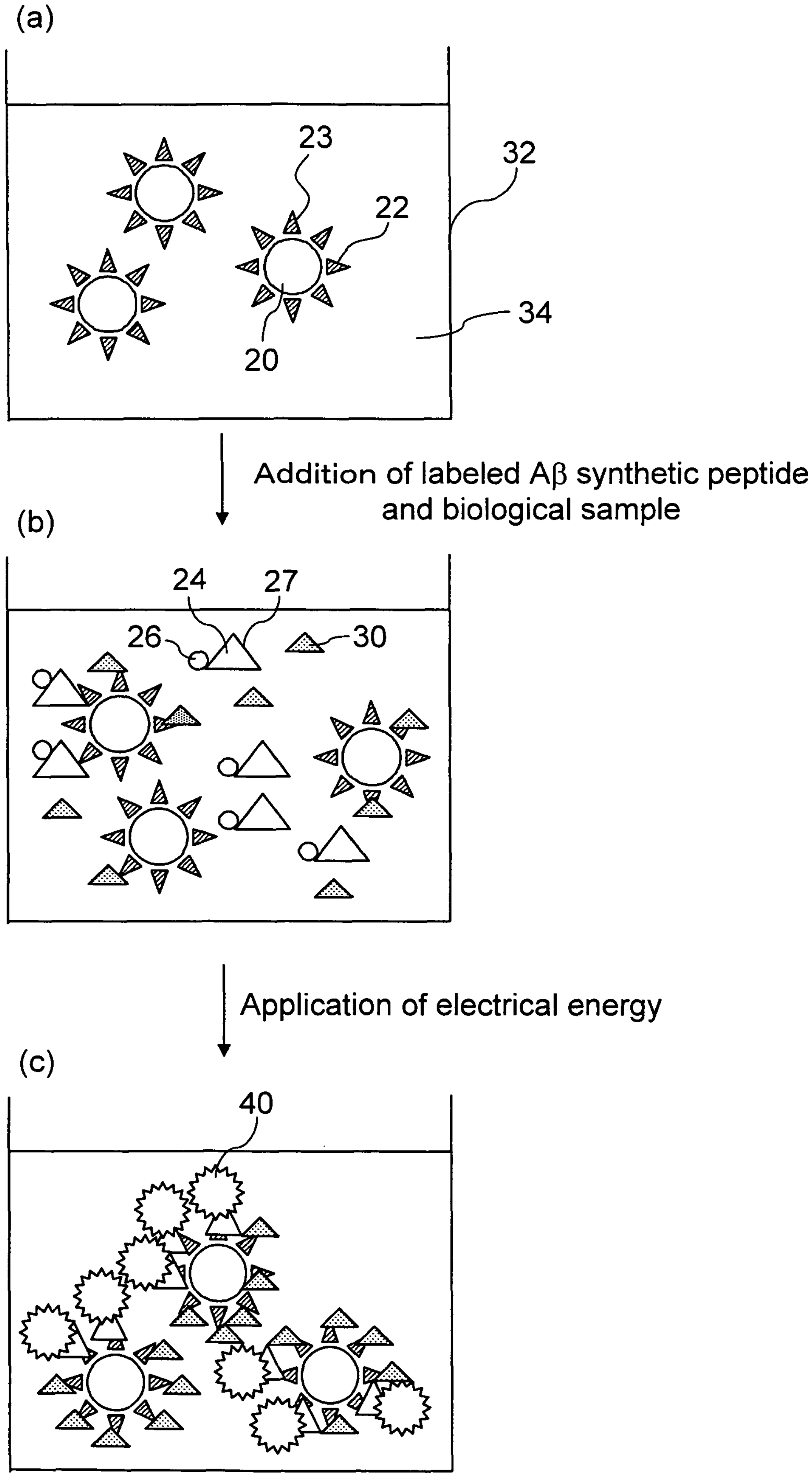
(a)
23
32
22
34
20
Addition of labeled Aβ synthetic peptide and biological sample
(b)
24
27
26
30
Application of electrical energy
(c)
40

FIG.3

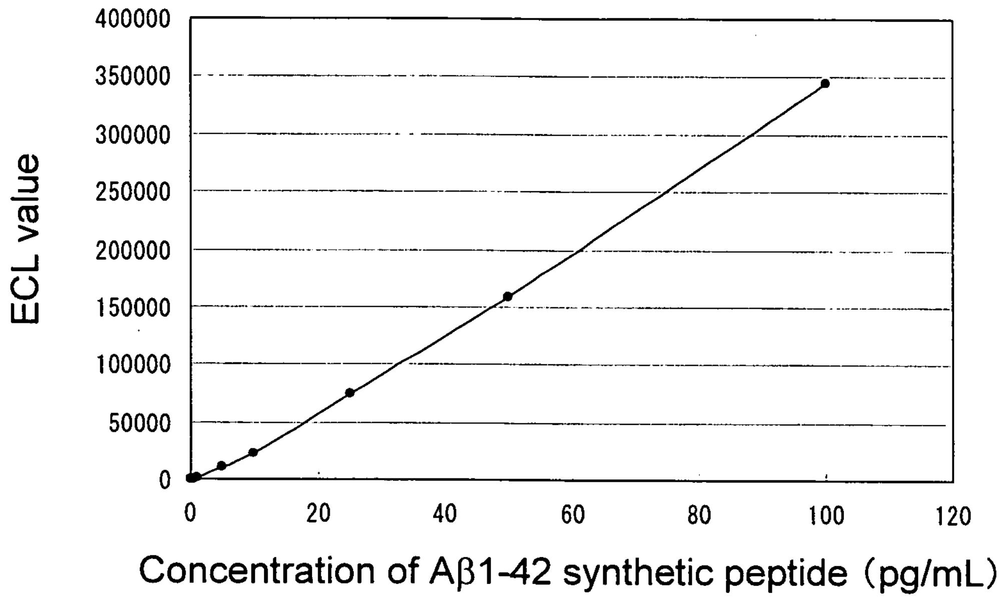
400000
350000
300000
250000
200000
150000
100000
50000
0
ECL value
0
20
40
60
80
100
120
Concentration of Aβ1-42 synthetic peptide (pg/mL)

FIG.4

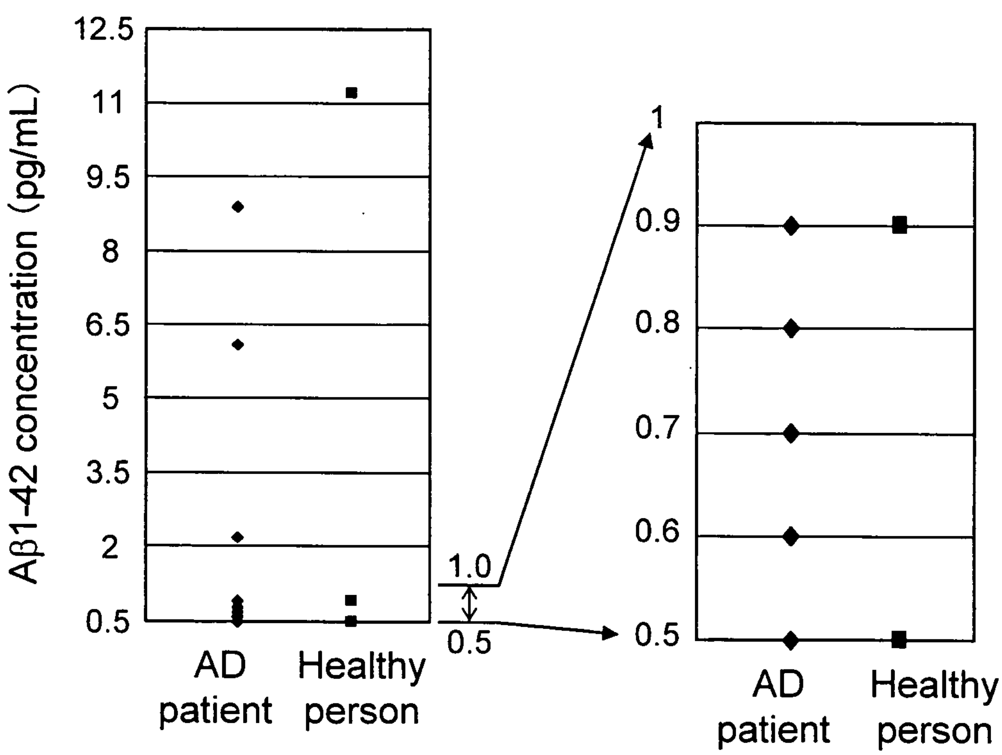
12.5
11
9.5
8
6.5
5
3.5
2
0.5
Aβ1-42 concentration (pg/mL)
AD patient
Healthy person
1.0
0.5
1
0.9
0.8
0.7
0.6
0.5
AD patient
Healthy person

# FIG.5

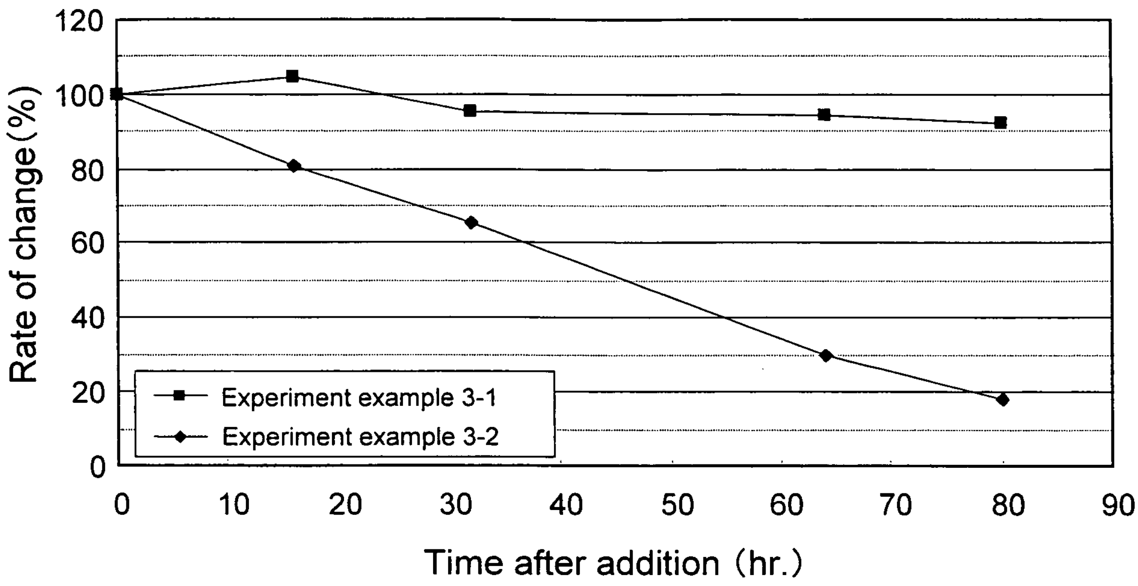
120
100
80
60
40
20
0
Rate of change(%)
0
10
20
30
40
50
60
70
80
90
Time after addition (hr.)
Experiment example 3-1
Experiment example 3-2

# FIG.6

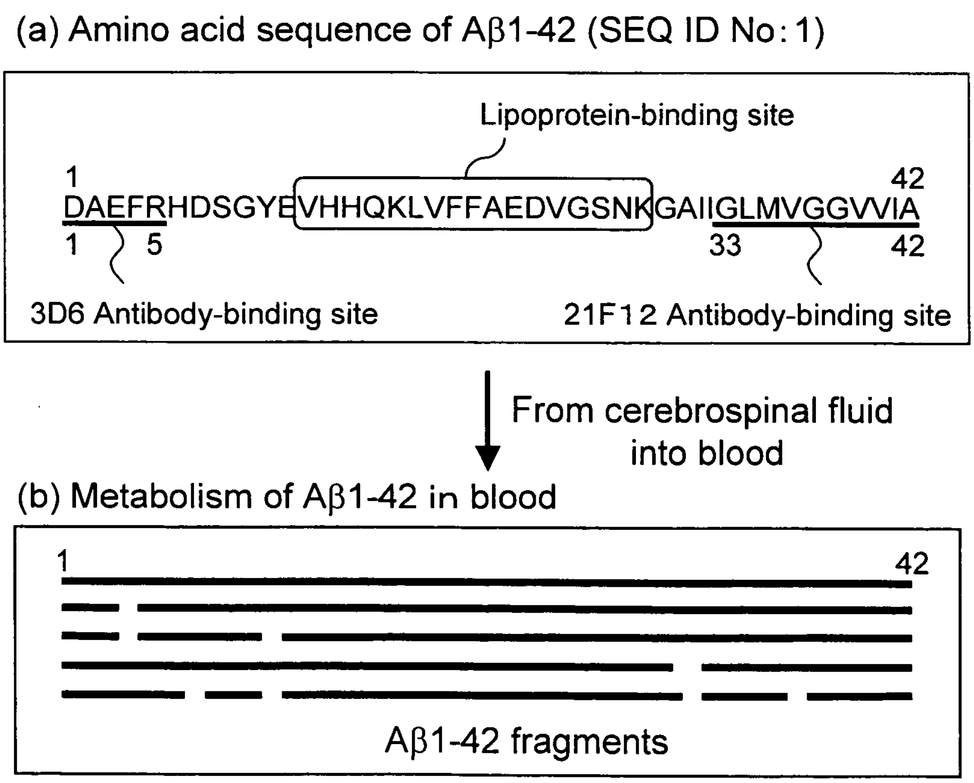
(a) Amino acid sequence of Aβ1-42 (SEQ ID No: 1)
Lipoprotein-binding site
1
42
DAEFRHDSGYEVHHQKLVFFAEDVGSNKGAIIGLMVGGVVIA
1
5
33
42
3D6 Antibody-binding site
21F12 Antibody-binding site
From cerebrospinal fluid into blood
(b) Metabolism of Aβ1-42 in blood
1
42
Aβ1-42 fragments

FIG.7

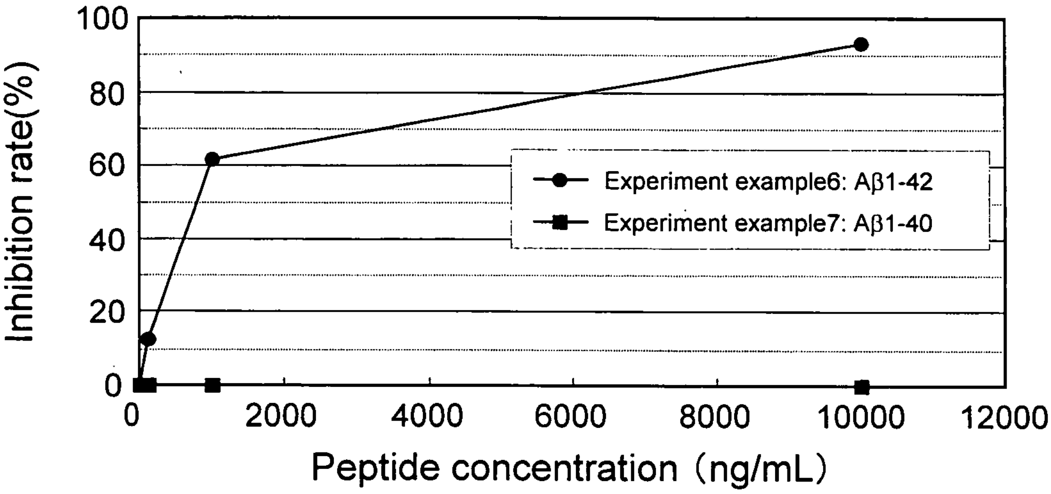
Inhibition rate(%)
100
80
60
40
20
0
0
2000
4000
6000
8000
10000
12000
Peptide concentration (ng/mL)
Experiment example6: Aβ1-42
Experiment example7: Aβ1-40

FIG.8

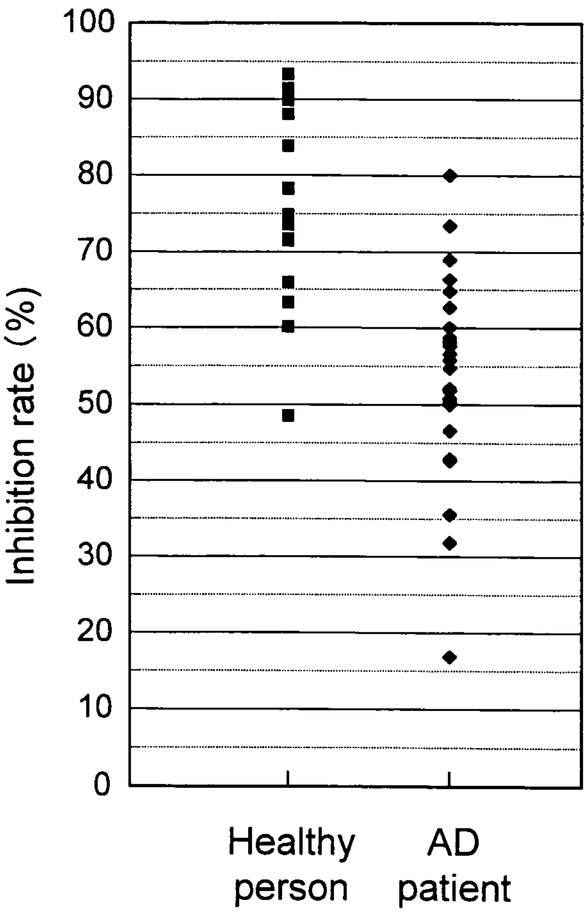
Inhibition rate (%)
100
90
80
70
60
50
40
30
20
10
0
Healthy person
AD patient

# FIG.9

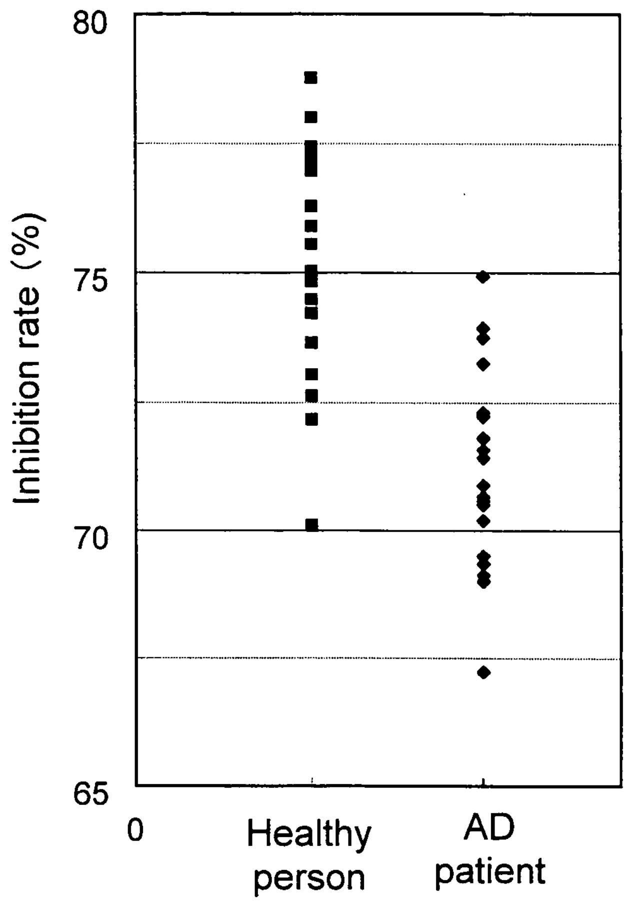
80
75
70
65
Inhibition rate (%)
0
Healthy person
AD patient

# FIG.10

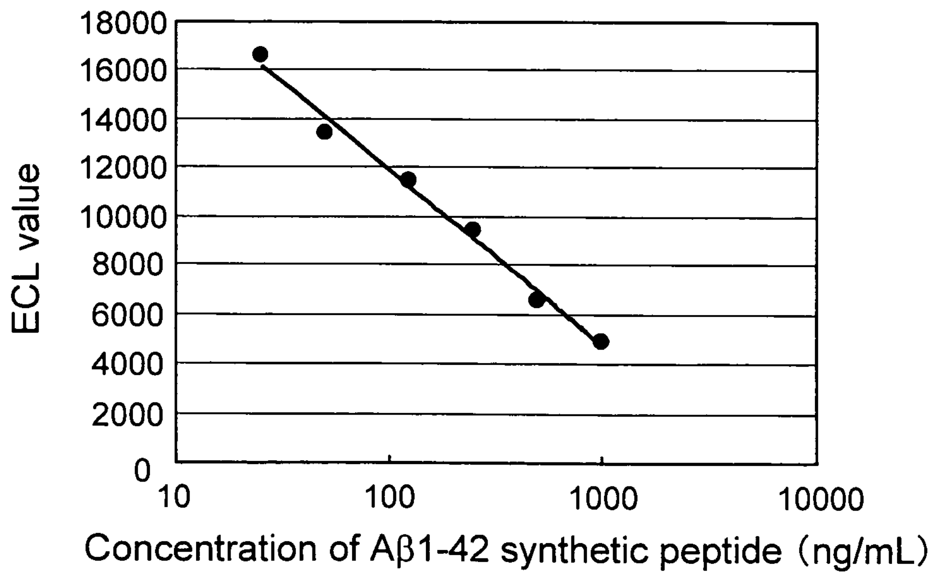
18000
16000
14000
12000
10000
8000
6000
4000
2000
0
ECL value
10
100
1000
10000
Concentration of Aβ1-42 synthetic peptide (ng/mL)

FIG.11

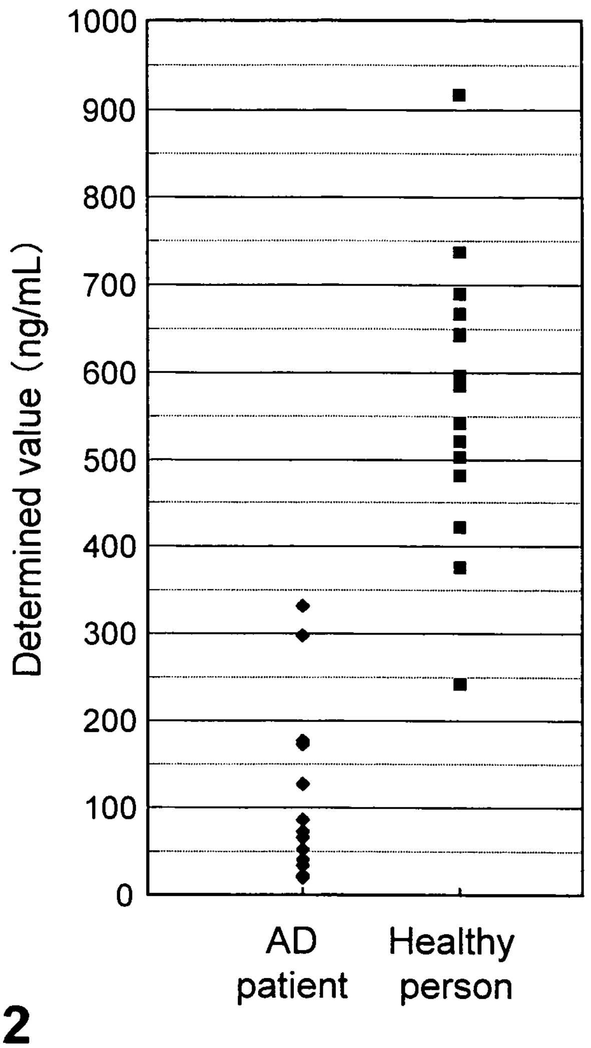
1000
900
800
700
600
500
400
300
200
100
0
Determined value (ng/mL)
AD patient
Healthy person

FIG.12

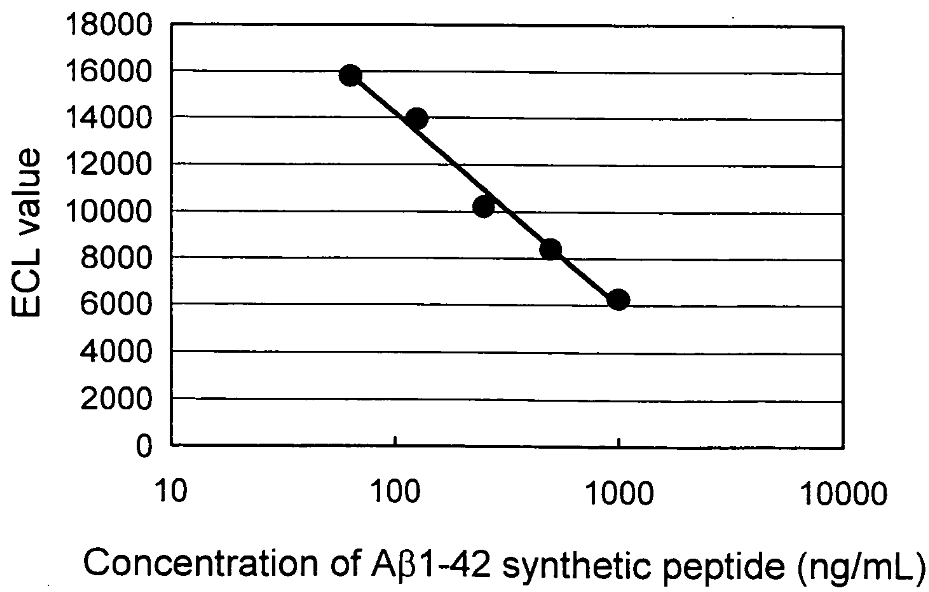
18000
16000
14000
12000
10000
8000
6000
4000
2000
0
ECL value
10
100
1000
10000
Concentration of Aβ1-42 synthetic peptide (ng/mL)

FIG.13

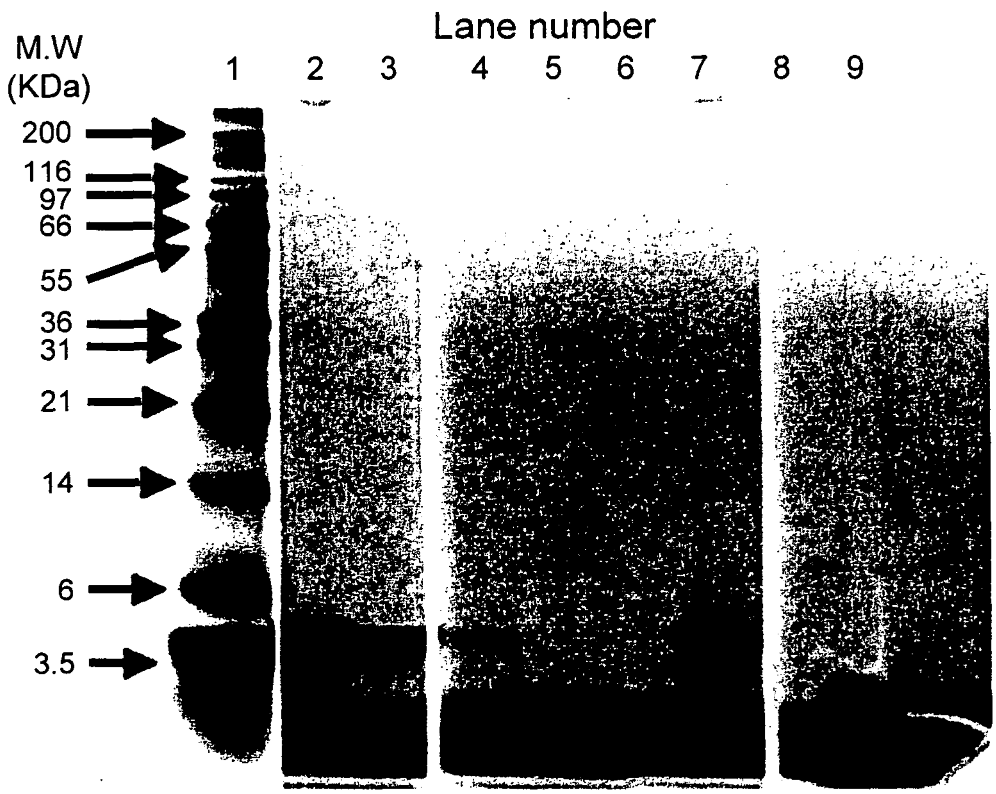


| Lane | Sample | Applied Aβ amount |
|---|---|---|
| 1 | Molecular marker | - |
| 2 | Aβ1-42 synthetic peptide | 500 ng |
| 3 | Aβ1-42 synthetic peptide | 50 ng |
| 4 | Aβ1-42 synthetic peptide | 10 ng |
| 5 | Aβ1-42 synthetic peptide | 5 ng |
| 6 | Aβ1-42 synthetic peptide | 100 ng |
| 7 | Experiment example 8-2 | 50 ng |
| 8 | Experiment example 8-3 | 50 ng |
| 9 | Experiment example 8-1 | 50 ng |

# FIG.14

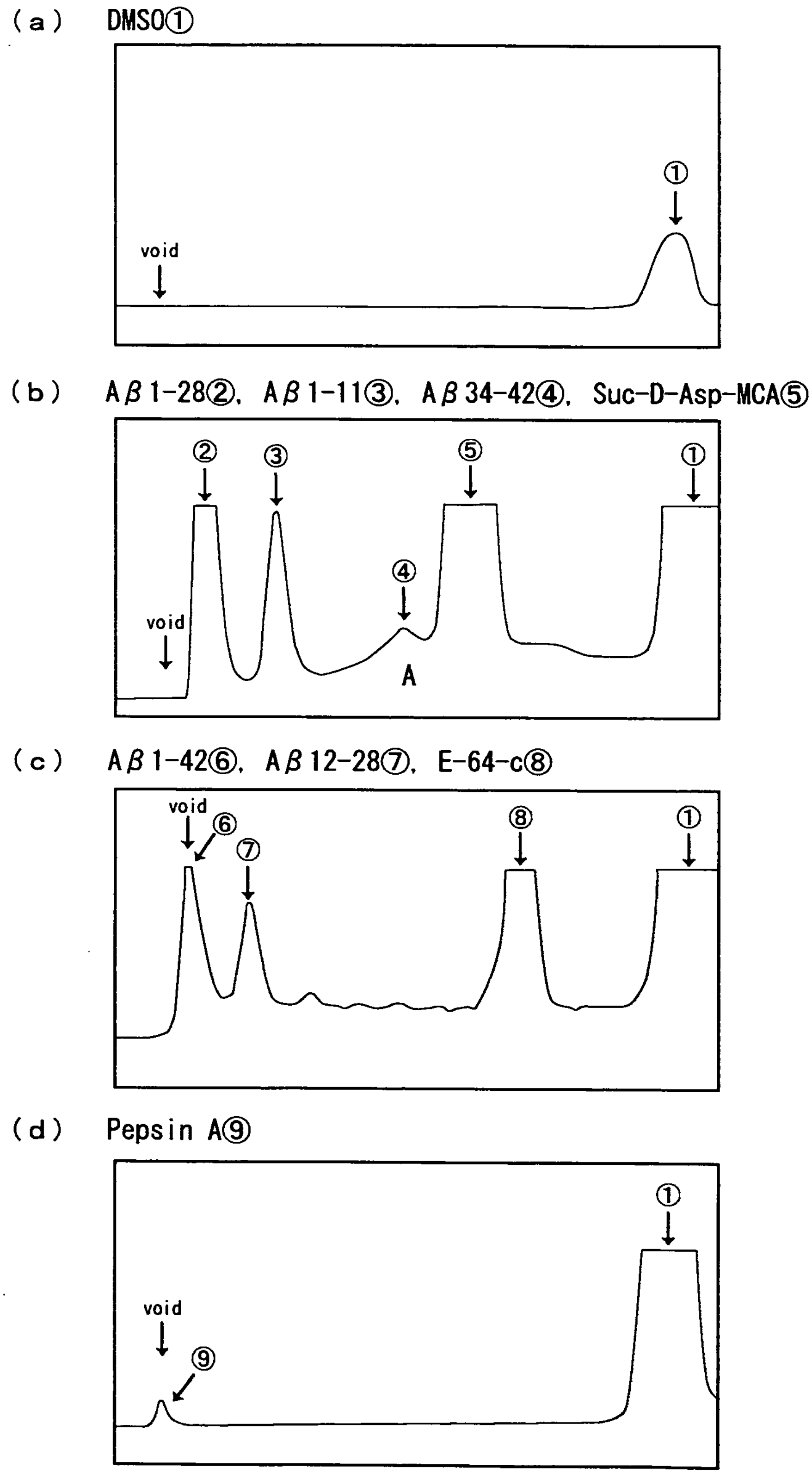
(a) DMSO①
void
①
(b) Aβ1-28②, Aβ1-11③, Aβ34-42④, Suc-D-Asp-MCA⑤
void
②
③
④
⑤
①
A
(c) Aβ1-42⑥, Aβ12-28⑦, E-64-c⑧
void
⑥
⑦
⑧
①
(d) Pepsin A⑨
void
⑨
①

# FIG.15

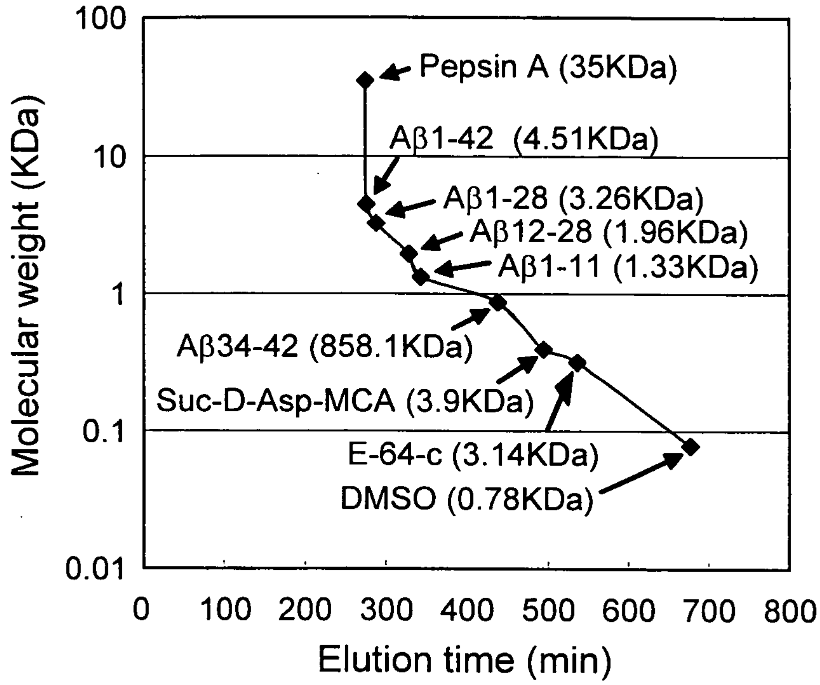

100
10
1
0.1
0.01
Molecular weight (KDa)
Pepsin A (35KDa)
Aβ1-42 (4.51KDa)
Aβ1-28 (3.26KDa)
Aβ12-28 (1.96KDa)
Aβ1-11 (1.33KDa)
Aβ34-42 (858.1KDa)
Suc-D-Asp-MCA (3.9KDa)
E-64-c (3.14KDa)
DMSO (0.78KDa)
0 100 200 300 400 500 600 700 800
Elution time (min)


# FIG.16

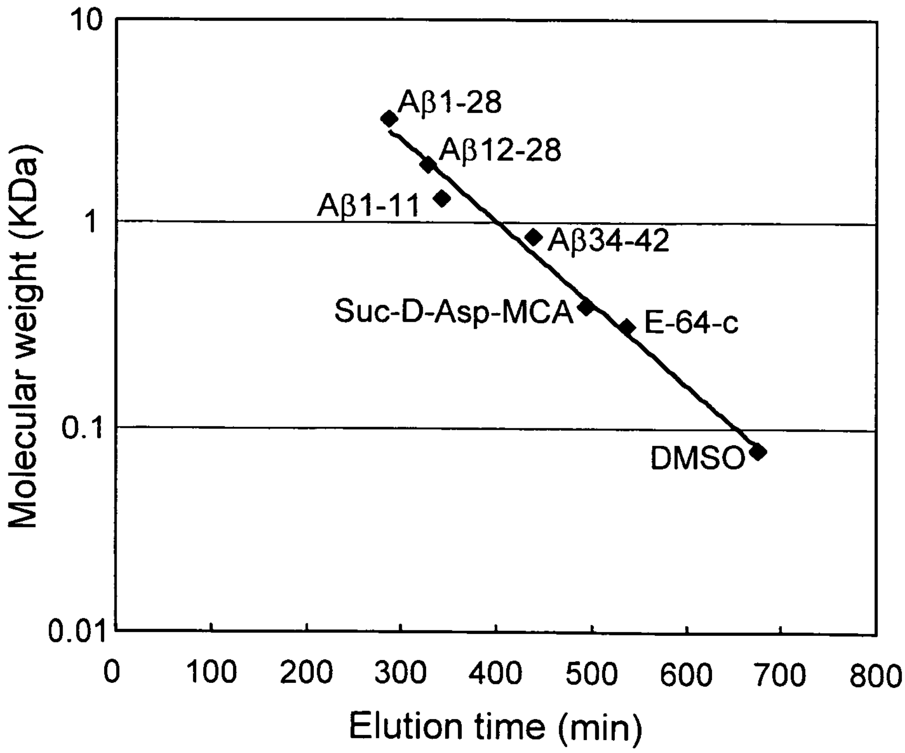

10
1
0.1
0.01
Molecular weight (KDa)
Aβ1-28
Aβ12-28
Aβ1-11
Aβ34-42
Suc-D-Asp-MCA
E-64-c
DMSO
0 100 200 300 400 500 600 700 800
Elution time (min)

FIG.17

DMSO①, Aβ1-28②, Aβ1-11③, Suc-D-Asp-MCA⑤

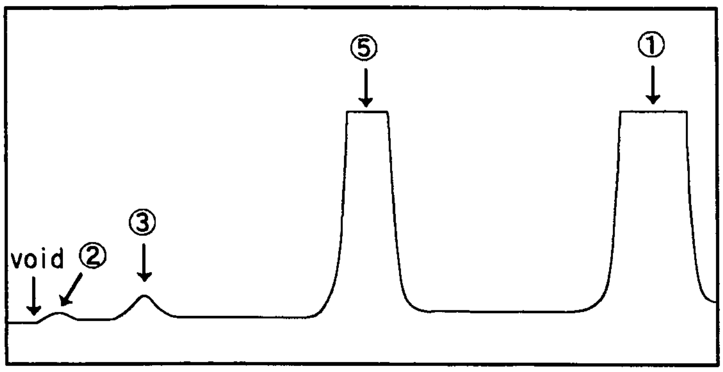


FIG.18

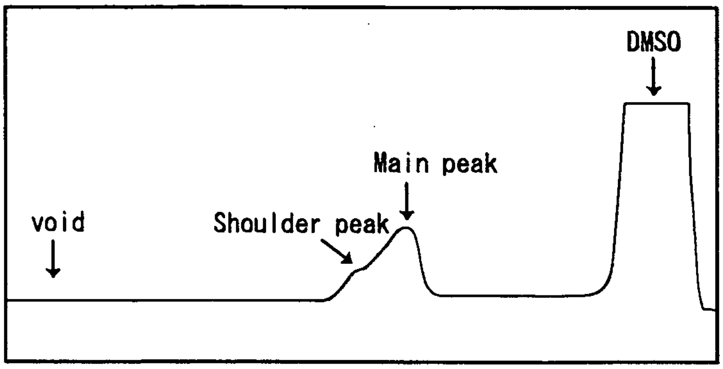

## INTERNATIONAL SEARCH REPORT

International application No.
PCT/JP2005/019787

A. CLASSIFICATION OF SUBJECT MATTER
*G01N33/543*(2006.01), *G01N33/53*(2006.01), *G01N33/541*(2006.01)

According to International Patent Classification (IPC) or to both national classification and IPC

B. FIELDS SEARCHED

Minimum documentation searched (classification system followed by classification symbols)
*G01N33/543*(2006.01), *G01N33/53*(2006.01), *G01N33/541*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | Hugo Vanderstichele et al., "Standardization of measurement of β-amyloid$_{(1-42)}$ in cerebrospinal fluid and plasma", Amyloid, Vol.7, pages 245 to 258, (2000) | 1,3,4,5,12, 13,20-25<br>2,6-11,14-19 |
| A | Akira TAMAOKA et al., "Amyloid β protein in plasma from patients with sporadic Alzheimer's disease", JOURNAL OH THE NEUROLOGICAL SCIENCES, 151, pages 65 to 68, (1996) | 1-25 |
| A | JP 3-151893 A (N.V. Innogenetics S.A.), 28 June, 1991 (28.06.91)<br>& EP 411974 A & IL 94912 A<br>& CA 2020544 A | 1-25 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>21 February, 2006 (21.02.06) | Date of mailing of the international search report<br>28 February, 2006 (28.02.06) |
| Name and mailing address of the ISA/<br>Japanese Patent Office<br>Facsimile No. | Authorized officer<br><br>Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9417197 A
- WO 9625435 A


### Non-patent literature cited in the description


- **TAMAOKA A et al.** *J. Neurol. Sci.,* 1997, vol. 148, 41-45
- **ANDREASEN N et al.** *Arch. Neurol.,* 1999, vol. 56, 673-680
- **GALASKO D et al.** *Arch. Neurol.,* 1998, vol. 55, 937-945
- **MOTTER R et al.** *Ann. Neurol.,* 1995, vol. 38, 643-648
- **MAYEUX R.** *Ann. Neurol.,* 1999, vol. 46, 412-416
- **MEHTA PD.** *Arch. Neurol.,* 2000, vol. 57, 100-105
- **TAMAOKA A et al.** *J. Neurol. Sci.,* 1996, vol. 141, 65-68
- **VANDERSTICHELE H et al.** *Amyloid,* 2000, vol. 7, 245-258
- **FUKUMOTO H et al.** *Arch. Neurol.,* 2003, vol. 60, 958-964
- **SUZUKI N et al.** *Science,* 1994, vol. 264, 1336-1340
- **CHAN W et al.** *Biochemistry,* 1996, vol. 35, 7123-1230
- **BIERE AL et al.** *J. Biol. Chem.,* 1996, vol. 271, 32916-32922
- **KUO YM et al.** *Biochem. Biophys. Res. Commun.,* 2000, vol. 268, 750-756
- **NANIWA et al.** An investigation of sensitive immunological assays with electrochemiluminescence (ECL. *JJCLA,* 1996, vol. 21 (5
- **TERO T et al.** *Neurobiology of Aging,* 2000, vol. 21, 735-740
- **KANAI M et al.** *Ann Neurol,* 1998, vol. 44, 17-26
- **SCHRÖDER J et al.** *Mol Psychiatry,* 1997, vol. 2, 505-7
- **CHRISTOPHER C.T. SMITH et al.** *Neuroscience Letters,* 2004, vol. 362, 48-50
- **G. ZHU et al.** *J. Pharm. Biomed. Anal.,* 2000, vol. 24, 281-290
- **KAYLER ; MILLSTEIN.** *Nature,* 1975, vol. 256, 495-497

SEQUENCE LISTING

```
<110>  Sanko Junyaku Co., Ltd.

<120>  Method for examining Alzheimer's disease and diagnostic reagent

<130>  EP51137FZ163pau

<140>  EP 05 799 253.9
<141>  2005-10-27

<150>  PCT/JP2005/19787
<151>  2005-10-27

<150>  JP 2004-314639
<151>  2004-10-28

<150>  JP 2005-052003
<151>  2005-02-25

<160>  2

<170>  PatentIn version 3.1

<210>  1
<211>  42
<212>  PRT
<213>  Homo sapiens

<400>  1

Asp Ala Glu Phe Arg His Asp Ser Gly Tyr Glu Val His His Gln Lys
1               5                   10                  15


Leu Val Phe Phe Ala Glu Asp Val Gly Ser Asn Lys Gly Ala Ile Ile
            20                  25                  30


Gly Leu Met Val Gly Gly Val Val Ile Ala
        35                  40


<210>  2
<211>  25
<212>  PRT
<213>  Artificial

<220>
<223>  Description of Artificial Sequence; Synthetic

<400>  2

Lys Lys Lys Lys Lys Lys Lys Lys Lys Lys Lys Lys Lys Lys Lys Gly
1               5                   10                  15


Leu Met Val Gly Gly Val Val Ile Ala
            20                  25
```